(19) Europäisches Patentamt
European Patent Office
Office européen des brevets

(11) Numéro de publication : **0 018 342**
**B1**

(12) **FASCICULE DE BREVET EUROPÉEN**

(45) Date de publication du fascicule du brevet : **16.02.83**

(21) Numéro de dépôt : **80870016.5**

(22) Date de dépôt : **04.03.80**

(51) Int. Cl.[3] : **C 07 C 69/30**, C 07 C 69/74, C 07 C 67/08, C 07 C 67/14, C 07 D317/24, C 07 D303/16, C 07 D317/72, A 61 K 31/215, A 61 K 31/22// C07C43/178, C07D317/20

(54) **Nouveaux dérivés du glycérol, leurs procédés de préparation ainsi que les compositions pharmaceutiques les contenant.**

(30) Priorité : **06.03.79 GB 7907932**

(43) Date de publication de la demande : **29.10.80 Bulletin 80/22**

(45) Mention de la délivrance du brevet : **16.02.83 Bulletin 83/07**

(84) Etats contractants désignés : **AT BE CH DE FR GB IT LU NL SE**

(56) Documents cités :
**DE A 1 289 620**
**DE A 2 202 535**
**FR A 2 147 091**

(73) Titulaire : **SANOFI, société anonyme**
**40, Avenue George V**
**F-75008 Paris (FR)**

(72) Inventeur : **Chignac, Michel**
**Le Gand Avenue du Lac**
**F-04200 Sisteron (FR)**
Inventeur : **Grain, Claude**
**Villa Les Olivettes La Croix du Sud**
**F-04290 Volonne (FR)**
Inventeur : **Jammot, Fernand**
**Les Egatiéres**
**F-04200 Sisteron (FR)**
Inventeur : **Pigerol, Charles**
**Rue Carnot, 8**
**F-93400 Saint-ouen (FR)**
Inventeur : **Eymard, Pierre**
**Rue de la Liberté, 22**
**F-38600 Fontaine (FR)**
Inventeur : **Ferrandes, Bernard**
**Rue Fantin Latour**
**F-38640 Claix (FR)**

(74) Mandataire : **Cauchie, Daniel et al**
**c/o S.A. LABAZ-SANOFI N.V. Avenue De Béjar, 1**
**B-1120 Bruxelles (BE)**

**EP 0 018 342 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Nouveaux dérivés du glycérol, leurs procédés de préparation ainsi que les compositions pharmaceutiques les contenant

La présente invention se rapporte à de nouveaux dérivés du glycérol doués d'activité pharmacologique, aux procédés de préparation de ces dérivés ainsi qu'aux compositions pharmaceutiques et vétérinaires les contenant.

Les nouveaux dérivés de l'invention consistent en des mono-, di- et triesters du glycérol correspondant à la formule générale

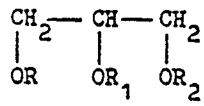

(I)

dans laquelle R, $R_1$ et $R_2$, qui sont identiques ou différents, représentent chacun hydrogène ou un radical acyle à savoir di-n-propylacétyle, tri-n-propylacétyle, éthyl-2 hexanoyle, n-propyl-4 heptanoyle, n-propyl-5 octanoyle, méthyl-2 éthyle-2 hexanoyle, méthyl-1 cyclohexylcarbonyle ou di-n-propylméthoxyacétyle à condition que R, $R_1$ et $R_2$ ne soient pas simultanément hydrogène ou di-n-propylacétyle.

Comme exemples de composés inclus dans le cadre de la présente invention, on peut citer les esters énumérés ci-dessous :

(Di-n-propylacétate)-1 de glycéryle
(Tri-n-propylacétate)-1 de glycéryle
(Ethyl-2 hexanoate)-1 de glycéryle
(Méthyl-1 cyclohexylcarboxylate)-1 de glycéryle
(Méthyl-2 éthyl-2 hexanoate)-1 de glycéryle
(Di-n-propylacétate)-2 de glycéryle
Bis-(di-n-propylacétate)-1,2 de glycéryle
Bis-(éthyl-2 hexanoate)-1,2 de glycéryle
Bis-(méthyl-1 cyclohexylcarboxylate)-1,2 de glycéryle
Bis-(tri-n-propylacétate)-1,2 de glycéryle
Bis-(méthyl-2 éthyl-2 hexanoate)-1,2 de glycéryle
Bis-(di-n-propylacétate)-1,3 de glycéryle
(Di-n-propylacétate)-1 (méthyl-1 cyclohexylcarboxylate)-3 de glycéryle
(Di-n-propylacétate)-1 bis-(méthyl-1 cyclohexylcarboxylate)-2,3 de glycéryle
(Di-n-propylacétate)-1 (méthyl-2 éthyl-2 hexanoate)-3 de glycéryle
(Di-n-propylacétate)-1 (méthyl-1 cyclohexylcarboxylate)-2 de glycéryle
Tri-(méthyl-1 cyclohexylcarboxylate)-1,2,3 de glycéryle
(Di-n-propylméthoxyacétate)-1 de glycéryle
(n-Propyl-4 heptanoate)-1 de glycéryle
(n-Propyl-5 octanoate)-1 de glycéryle.

Comme il sera décrit en détail par la suite, on a trouvé que des mélanges convenables d'esters de l'invention possèdent des qualités qui les rendent particulièrement utiles, ces mélanges pouvant être considérés également comme de nouveaux composés.

En conséquence, l'invention se rapporte, en outre, à des mélanges d'esters de l'invention, par exemple des mélanges de bis-(di-n-propylacétate)-1,2 de glycéryle/bis-(di-n-propylacétate)-1,3 de glycéryle, des mélanges de bis-(di-n-propylpropionate)-1,2 de glycéryle/bis-(di-n-propylpropionate)-1,3 de glycéryle et des mélanges de bis-(di-n-propylméthoxyacétate)-1,2 de glycéryle/bis-(di-n-propylméthoxyacétate)-1,3 de glycéryle.

Les composés de l'invention possèdent des centres d'isomérie et peuvent donc être produits sous forme d'isomères optiques, d'isomères de position ou de mélanges de ces isomères.

Si on le désire, on peut séparer ces isomères de leur mélange aux étapes appropriées de la préparation de ces mélanges par des méthodes connues en soi pour obtenir les isomères individuels désirés.

Ces isomères, ainsi que les mélanges de ces isomères font également partie de la présente invention.

Un autre objet de l'invention se rapporte à une composition pharmaceutique ou vétérinaire comprenant, comme principe actif essentiel, au moins un ester de l'invention en association avec un véhicule pharmaceutique ou un excipient approprié.

Comme on le décrira plus en détail par la suite, on a trouvé que les esters de l'invention sont doués de propriétés biochimiques et pharmacologiques susceptibles de les rendre particulièrement utiles dans le traitement d'états pathologiques dus à un dérèglement du système nerveux central ainsi que de troubles relevant plus particulièrement de la neuropsychiatrie.

Chez l'être humain, les composés de l'invention pourront être administrés journellement à raison de 10 mg/kg à 50 mg/kg.

Les documents DE-A-1 289 620, FR-A-2 147 091 et DE-A-2 202 535 se rapportent notamment à des esters d'acides monocarboxyliques du glycérol ou de propanediol. Mais ces esters ainsi que leurs propriétés sont différents des composés de la présente invention.

Les composés de l'invention peuvent être préparés de différentes façons eu égard à leur structure chimique.

I. Monoesters

A. α-Monoesters

Ces esters, qui peuvent être représentés par la formule générale

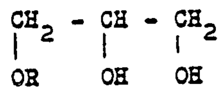

(ii)

dans laquelle R représente un radical acyle tel que défini dans la formule I, peuvent être préparés en faisant réagir, sous reflux, le glycérol et une cétone de formule générale

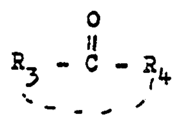

(III)

dans laquelle $R_3$ représente méthyle, $R_4$ représente méthyle, éthyle, méthyl-2 butyle ou $R_3$ et $R_4$, lorsqu'il sont pris ensemble, représentent un radical pentaméthylène, la réaction ayant lieu en présence d'acide p-toluènesulfonique comme catalyseur et éventuellement en présence d'un solvant tel que le benzène pour obtenir un cétal de formule générale

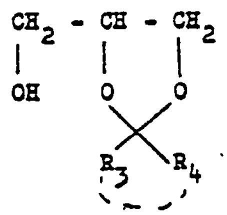

(IV)

dans laquelle $R_3$ et $R_4$ ont la même signification que précédemment.

Le composé de formule IV ainsi obtenu est ensuite estérifié dans la pyridine ou dans un mélange de diméthylformamide/pyridine à 65 °C ± 5 °C avec le chlorure d'un acide de formule générale

$$R—OH \qquad V$$

dans laquelle R représente un radical acyle tel que défini dans la formule I, pour obtenir un cétal de formule générale

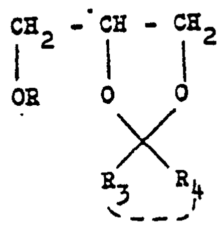

(VI)

dans laquelle R représente un radical acyle tel que défini dans la formule I et $R_3$ et $R_4$ ont la même signification que précédemment, qui est ensuite hydrolysé à la température ambiante, dans un milieu hydrométhanolique et en présence d'un acide inorganique dilué tels que l'acide chlorhydrique, l'acide sulfurique ou l'acide acétique, pour donner les composés requis de l'invention.

Lorsque le chlorure de l'acide de formule V est encombré, le cétal de formule IV sera, au préalable, mis en réaction avec le sodium ou l'hydrure de sodium dans le N,N-diméthylformamide et l'estérification sera effectuée dans le N,N-diméthylformamide ou un mélange de N,N-diméthylformamide/hexaméthylènephosphoramide à une température comprise entre 40° et 90 °C.

B. β-Monoesters

Ces esters, qui peuvent être représentés par la formule générale

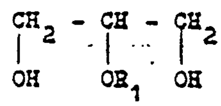

(VII)

dans laquelle $R_1$ représente un radical acyle tel que défini dans la formule I, peuvent être préparés en faisant réagir le glycérol avec la benzaldéhyde en présence d'un entraîneur d'eau et d'acide p-toluènesulfonique comme catalyseur, pour obtenir un mélange d'environ 90/10 de dérivé benzylidène-1,2 glycérol et benzylidène-1,3 glycérol.

Lorsque ce mélange est maintenu à faible température, c'est-à-dire aux environs de 0 °C et en présence d'acide chlorhydrique sec, le dérivé benzylidène-1,2 s'isomérise en dérivé benzylidène-1,3.

Le dérivé benzylidène-1,3 en question, est alors estérifié avec le chlorure d'un acide de formule V à la température de 60 °C ± 5 °C et en présence d'un accepteur d'acide tel que la pyridine pour donner un dérivé benzylidène de formule générale

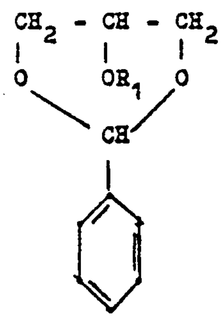

(VIII)

dans laquelle $R_1$ a la même signification que dans la formule VII, lequel est alors mis en réaction avec l'hydrogène dans un milieu alcoolique, par exemple l'éthanol, et en présence de charbon palladié comme catalyseur, la réaction étant effectuée à la pression atmosphérique et à la température ambiante, pour donner le composé requis de l'invention. Au lieu de benzaldéhyde, on peut également utiliser un dérivé de celui-ci substitué sur l'entité aromatique.

II. Diesters

Ces esters, qui peuvent être représentés par la formule générale

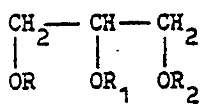

(IX)

dans laquelle un des symboles R, $R_1$ et $R_2$ représente hydrogène et les deux autres représentent un radical acyle tel que défini dans la formule I, peuvent être obtenus comme suit :

A. Diesters-1,2

a) Lorsque R représente hydrogène et $R_1$ et $R_2$ sont identiques et représentent un radical acyle, en faisant réagir un cétal de formule IV avec le chlorure de benzyle en présence d'un métal alcalin, par exemple le sodium, pour obtenir l'éther benzylique de formule générale

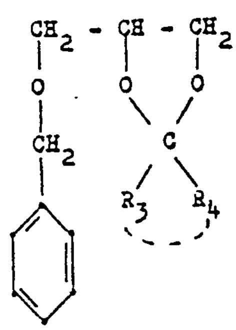

(X)

dans laquelle $R_3$ et $R_4$ ont la même signification que précédemment, la réaction étant effectuée sous

reflux dans un solvant approprié tel que le toluène.

L'éther benzylique ainsi obtenu est ensuite hydrolysé dans un mélange eau/acide acétique à la température de reflux de ce mélange ou dans un mélange hydrométhanolique/acide chlorhydrique à température ambiante, ce qui fournit le benzyl-1 glycérol qui est ensuite estérifié avec le chlorure d'un acide de formule V en présence d'un accepteur d'acide tel que la pyridine et à température comprise entre 50 et 95 °C pour obtenir un diester de formule générale

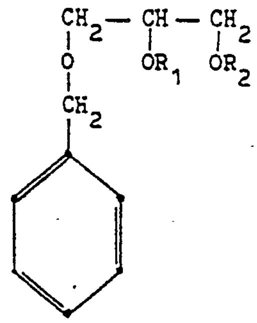

(XI)

dans laquelle $R_1$ et $R_2$ sont identiques et représentent un radical acyle tel que défini dans la formule I, lequel est ensuite mis en réaction avec l'hydrogène dans un alcool tel que l'éthanol en présence de charbon palladié comme catalyseur, la réaction étant effectuée à pression atmosphérique et à température ambiante, ce qui fournit les composés requis de l'invention.

Lorsque l'un ou l'autre des radicaux $R_1$ et $R_2$ est encombré, le composé de formule X sera préparé de préférence après sodation du benzyl-1 glycérol avec l'hydrure de sodium, l'estérification étant effectuée dans le N,N-diméthylformamide.

Les diesters-1,2 de glycérol de l'invention dans lesquels les radicaux situés en positions-1 et -2 sont identiques peuvent également être préparés selon d'autres procédés.

D'autres méthodes ont, en effet, été expérimentées dans le cas particulier du bis-(di-n-propylacétate)-1,2 de glycéryle.

Par exemple, ce diester peut également être obtenu en faisant réagir l'épichlorhydrine avec un sel de sodium, dans un solvant approprié tel que par exemple l'eau ou un mélange toluène/diméthylformamide et à une température comprise entre 60° et 80 °C pour obtenir le di-n-propylacétate d'époxy-2,3 propyle. Ce dérivé époxy est ensuite mis en réaction avec l'acide di-n-propylacétique à une température comprise entre 60° et 80 °C en présence de trifluorure de bore, de préférence sous la forme de son éthérate, ou en présence d'acide sulfurique, ce qui fournit le diester requis.

b) Lorsque R représente hydrogène et $R_1$ et $R_2$ sont différents et représentent un radical acyle en faisant réagir l'épichlorhydrine avec le dérivé de métal alcalin, par exemple le dérivé sodique, d'un acide de formule V, la réaction étant effectuée dans un solvant approprié tel qu'un mélange toluène/diméthylformamide et à une température comprise entre 60° et 80 °C pour obtenir un dérivé époxy de formule générale

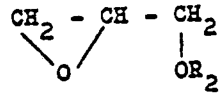

(XII)

dans laquelle $R_2$ représente un radical acyle tel que défini dans la formule I.

Ce dérivé époxy est alors mis en réaction à une température comprise entre 60° et 80 °C avec un acide différent de formule V en présence de trifluorure de bore, de préférence sous la forme de son éthérate ou en présence d'acide sulfurique, ce qui fournit l'ester requis de l'invention.

B. Diesters-1,3

a) Lorsque R et $R_2$ sont identiques et représentent un radical acyle et $R_1$ représente hydrogène, en estérifiant la dihydroxyacétone avec le chlorure d'un acide de formule V, cette réaction étant effectuée au reflux, en présence d'un accepteur d'acide tel que la pyridine et dans un solvant tel que l'éther isopropylique, pour obtenir un cétodiester de formule générale

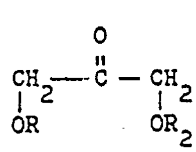

(XIII)

dans laquelle R et $R_2$ représentent un radical acyle tel que défini dans la formule I, lequel est ensuite réduit, à température ambiante avec le borohydrure de potassium dans un éther tel que le tétrahydrofuranne. Le complexe ainsi formé est alors hydrolysé avec un acide fort inorganique tel que par exemple l'acide chlorhydrique pour donner le composé requis de l'invention.

b) Lorsque R et $R_2$ sont différents et représentent un radical acyle et $R_1$ représente hydrogène, en estérifiant un diol de formule générale

```
CH2 - CH - CH2
|     |    |
OR    OH   OH
```
(XIV)

dans laquelle R représente un radical acyle tel que défini dans la formule I, avec le chlorure d'un acide de formule V, la réaction étant effectuée entre la température ambiante et 110 °C et dans un accepteur d'acide tel que la pyridine pour obtenir le composé requis de l'invention.

On fera réagir de préférence 1 à 1,5 équivalent de chlorure d'acide de formule V avec 1 équivalent de diol de formule XIV.

Des mélanges particulièrement utiles d'esters de l'invention contenant un pourcentage élevé de diesters de l'invention peuvent être obtenus selon des procédés spécifiques.

Dans le cas particulier d'esters di-n-propylacétiques du glycérol, la méthode suivante peut être utilisée.

L'estérification, à une température comprise entre − 5 °C et + 10 °C d'un équivalent de glycérol avec deux équivalents d'acide di-n-propyl acétique ou deux équivalents de son chlorure, en présence de pyridine comme accepteur d'acide, suivie d'une rectification du mélange d'esters ainsi obtenu par distillation sous haut vide, par centrifugation moléculaire ou par chromographie en phase gazeuse fournit un mélange d'esters d'acide di-n-propylacétique et de glycérol avec un rendement de 73 à 74 %.

Ce mélange comprend :

| | |
|---|---|
| Monoesters d'acide di-n-propylacétique | ≤ 10 % |
| Diesters-1,2 et -1,3 d'acide di-n-propylacétique | ≥ 88 % |
| Triester-1,2,3 d'acide di-n-propylacétique, acide di-n-propylacétique, glycérol | ≤ 2 % |

et sera dénommé, par la suite, « Mélange A ».

Cependant, on a découvert de manière surprenante que des mélanges de bis-(di-n-propylacétate)-1,2 de glycéryle/bis-(di-n-propylacétate)-1,3 de glycéryle peuvent être obtenus pratiquement exempts de mono- et de tri-esters, en estérifiant dans le N,N-diméthylformamide comme solvant, à une température comprise entre 100 et 110 °C et pendant 5 à 10 heures, un équivalent de dichloro-1,3 propanol-2 avec deux équivalents, de préférence en excès, d'un sel de métal alcalin, de préférence le sel de sodium, d'acide di-n-propylacétique puis en rectifiant le mélange d'esters ainsi obtenu par exemple par distillation sous haut vide, centrifugation moléculaire ou chromatographie en phase gazeuse de façon à obtenir un mélange des diesters correspondants-1,2 et -1,3 du glycérol avec un rendement supérieur à 85 %.

Selon des conditions préférées de réaction, on obtient le mélange de tels diesters-1,2 et -1,3 du glycérol en faisant réagir à la température de 100 °C, pendant 8 heures et dans le N,N-diméthylformamide, le dichloro-1,3 propanol-2 avec deux équivalents de di-n-propylacétate de sodium, de préférence en excès d'environ 5 %, puis en rectifiant le mélange de diesters ainsi obtenu, par exemple par distillation sous haut vide, centrifugation moléculaire ou chromatographie en phase gazeuse, ce qui fournit un mélange de bis-(di-n-propylacétate)-1,2 et -1,3 de glycéryle avec un rendement d'environ 88 à 95 %.

Lorsque de telles conditions de réaction sont respectées, on obtient un mélange reproductible de bis-(di-n-propylacétate)-1,3 de glycéryle/bis-(di-n-propylacétate)-1,2 de glycéryle dans un rapport de 1,43 : 1 à 1,54 : 1.

Ce mélange comprend :

| | |
|---|---|
| Monoester-1 de glycéryle | ≤ 1 % |
| Diesters-1,2 et -1,3 de glycéryle | ≥ 98 % |
| Triester-1,2,3 de glycéryle, acide di-n-propylacétique, dichloro-1,3 propanol-2 | ≤ 1 % |

et sera dénommé, par la suite, « Mélange B ».

Dans le procédé décrit précédemment, le N,N-diméthylformamide peut être utilisé en quantité trois à cinq fois la quantité totale des réactifs sans influencer le rendement total en mélange final de diesters.

De plus, le di-n-propylacétate sodique de départ peut être soit ce dernier sous forme solide, soit une solution aqueuse de di-n-propylacétate de sodium préparée « in situ » à partir d'acide di-n-propylacétique et une solution aqueuse à 50 % d'hydroxyde de sodium.

Il est évident que lorsqu'on utilise une solution aqueuse de di-n-propylacétate de sodium comme produit de départ, de l'eau est introduite dans le milieu réactionnel. Si le volume d'eau ainsi introduite n'est pas supérieur à 5 % du volume de N,N-diméthylformamide, le rendement en mélange final de diesters

n'est pas modifié.

Le procédé ainsi décrit pour préparer un mélange de bis-(di-n-propylacétates)-1,2 et -1,3 de glycéryle présente de nombreux avantages, le plus important étant l'obtention d'un mélange final isolé de grande pureté. Les produits bruts ne contiennent pas de triesters. Après une seule distillation, le contenu d'un tel mélange en diesters totaux est ≥ 98 %.

Par conséquent, le mélange de bis-(di-n-propylacétates)-1,2 et -1,3 de glycéryle, plus particulièrement le « Mélange B », présente une valeur commerciale incontestable : il est facile à préparer d'une manière reproductible, pratiquement exempt d'impuretés et de prix de revient modique puisqu'aucune séparation des constituants n'est requise.

En outre, ce mélange, de même que le « Mélange A », présente des intérêts pharmacologique et pharmacocinétique de première importance comme il en sera discuté par la suite.

Les procédés décrits ci-dessus pour la préparation de mélanges de bis-(di-n-propylacétates)-1,2 et -1,3 de glycéryle peuvent être appliqués également à la préparation d'autres mélanges de diesters de l'invention.

Les mélanges de mono-, di- et triesters ainsi obtenus peuvent être séparés, par la suite, au moyen, par exemple, d'une distillation sous haut vide, d'une centrifugation moléculaire ou de chromatographie en phase gazeuse, pour obtenir les esters désirés sous forme pure.

III. Triesters

Ces esters, qui peuvent être représentés par la formule générale

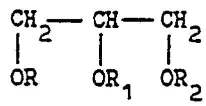

(XV)

dans laquelle R, $R_1$ et $R_2$ représentent chacun un radical acyle tel que défini dans la formule I peuvent être obtenus comme suit :

a) Lorsque R, $R_1$ et $R_2$ représentent des radicaux acyles identiques, en estérifiant le glycérol :
— soit avec un acide de formule V en excès à la température de reflux du milieu et en présence d'acide p-toluènesulfonique comme catalyseur
— soit avec le chlorure d'un acide de formule V en excès à une température de 60-70 °C et en présence d'un accepteur d'acide tel que la pyridine
de façon à obtenir le composé désiré de l'invention.

b) Lorsque R et $R_1$ représentent des radicaux acyles identiques et $R_2$ représente un radical acyle différent, en estérifiant un monoester de formule générale

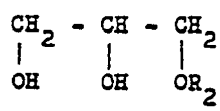

(XVI)

dans laquelle $R_2$ représente un radical acyle tel que défini dans la formule I, avec le chlorure d'un acide de formule V à température comprise entre la température ambiante et 110 °C et dans un accepteur d'acide tel que la pyridine, ce qui fournit les composés désirés de l'invention.

c) Lorsque R et $R_2$ représentent des radicaux acyles identiques et $R_1$, qui est différent, représente également un radical acyle, en estérifiant un diester de formule générale

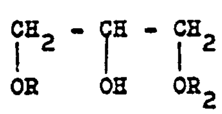

(XVII)

dans laquelle R et $R_2$, sont identiques et représentent un radical acyle tel que défini dans la formule I, avec le chlorure d'un acide de formule V en présence d'un accepteur d'acide tel que la pyridine et à température du reflux, de façon à obtenir le composé désiré de l'invention.

d) Lorsque R, $R_1$ et $R_2$ représentent tous différents radicaux acyles, en estérifiant un diester de formule générale

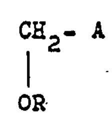

(XVIII)

dans laquelle R représente un radical acyle tel que défini dans la formule I et A représente un radical

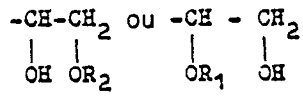

ou

dans lesquels $R_1$ et $R_2$ représentent chacun un radical acyle tel que défini dans la formule I, avec le chlorure d'un acide de formule V à la température de reflux du milieu et en présence d'un accepteur d'acide tel que la pyridine, ce qui fournit les composés désirés de l'invention.

Les chlorures des acides de formule V de départ peuvent être obtenus selon des procédés connus en faisant réagir l'acide correspondant avec le chlorure de thionyle.

De même, les sels de métaux alcalins de ces acides sont obtenus en faisant réagir les acides en question avec l'hydroxyde du métal alcalin correspondant.

Les acides en question sont soit des composés connus ayant été publiés dans les brevets N$^{os}$ 3.325.361, 3.958.011 et 4.025.649 des Etats-Unis d'Amérique ainsi que dans le brevet belge N° 875.882 ou des composés qui peuvent être obtenus par des procédés décrits dans ces brevets. Les composés de l'invention possèdent de remarquables propriétés biochimiques, en particulier en effet inhibiteur compétitif de la transaminase γ-aminobutyrique α-cétoglutarique ainsi que de précieuses propriétés neurotropes et psychotropes se manifestant à des degrés divers. Celles-ci comprennent, en particulier, des propriétés antianoxique, anticonvulsivante, anxiolytique et antipsychotique. Les composés de l'invention peuvent également agir comme régulateurs du système nerveux central et comme potentialisateurs des dépresseurs du système nerveux central.

L'ensemble de ces propriétés est susceptible de rendre les composés de l'invention particulièrement utiles pour le traitement de différentes variétés de troubles neurologiques centraux et de désordres relevant plus particulièrement de la neuropsychiatrie.

Comme exemples de tels troubles neurologiques centraux ou de désordres provoqués par un dérèglement du système nerveux central, on peut citer : des états et des crises convulsives tels que par exemple l'épilepsie, des états choréiques tels que la chorée de Hungtington, des troubles de la mémoire, de l'équilibre et de la vigilance ainsi que des névroses et psychoses d'origines diverses.

Des esters aliphatiques et alicycliques de diols ou de polyols sont connus ayant été décrits dans l'art antérieur.

De ce point de vue, on citera le brevet N° 1.289.620 de la République Fédérale d'Allemagne se rapportant à des diesters utiles dans le domaine dermatologique, ces diesters étant formés au départ d'alcools comprenant uniquement deux fonctions hydroxyles. De même, on mentionnera le brevet français N° 2.147.091 relatif notamment à des intermédiaires de synthèse chimique constitués d'α-monoesters de glycérol formés au départ d'acides comprenant au plus 7 atomes de carbone. En outre, on fera mention de la demande de brevet N° 2.202.535 de la République Fédérale d'Allemagne se rapportant notamment au monoester-1 et au diester-1,2 de glycérol et d'acide isopropyl-3 méthyl-6 cyclohexylcarboxylique utiles comme agents stimulants les récepteurs physiologiques sensibles au froid. Cependant, aucun de ces documents n'était susceptible de laisser prévoir les propriétés particulières mises en évidence chez les esters de la présente invention.

De même, on citera le brevet N° 3.686.238 des Etats-Unis d'Amérique lequel couvre des dérivés du glycérol ayant une activité exaltée et prolongée après administration orale.

Certains de ces dérivés du glycérol sont constitués par le glycérol dont un des hydroxyles est substitué par un reste médicamenteux alors qu'un ou les deux hydroxyles restants sont substitués par un acide gras naturel ayant de 8 à 24 atomes de carbone.

On a maintenant découvert de manière tout à fait surprenante, selon la présente invention, qu'une libération lente de l'entité médicamenteuse peut être obtenue à partir d'un ester formé au départ d'un acide thérapeutiquement actif de formule V ci-dessus et du glycérol, sans qu'il soit nécessaire de substituer au moins un des OH du glycérol par un acide gras naturel.

Cette invention ne pouvait en aucune façon être suggérée ou déduite de l'art antérieur.

Les composés de formule I ci-dessus représentent un progrès sur les esters de l'art antérieur puisqu'aucun acide gras naturel ne fait partie de la molécule des esters de l'invention. On peut, de cette manière, éliminer une étape dans la synthèse à savoir la substitution d'un ou de deux groupements hydroxyles du glycérol par un acide gras naturel. Les composés de l'invention présentent donc une structure plus simple que celle des composés de l'art antérieur.

Il apparaît, par conséquent, que le radical acyle gras faisant partie de la molécule des esters du brevet américain N° 3.686.238 n'offre aucun avantage particulier. Ce radical ne semble jouer qu'un rôle de véhicule au même titre que le glycérol supportant le reste médicamenteux.

En outre, on remarquera qu'aucun reste de médicament apparaissant dans le susdit brevet américain n'est inclus dans le cadre des acides de formule V ci-dessus.

Au surplus, aucune preuve de préparation ou d'efficacité des dérivés du brevet américain N° 3.686.238 n'y est citée alors que ce brevet couvre de manière générique des milliers de composés.

Ainsi, l'hydrolyse des esters de l'invention libère, chez l'animal et chez l'être humain, le glycérol et un acide thérapeutiquement actif correspondant à la formule générale V ci-dessus.

En conséquence, de cette hydrolyse, on a observé que les composés de l'invention peuvent être

considérés comme une forme à libération lente des acides en question permettant d'obtenir un taux thérapeutique prolongé chez l'animal et/ou chez l'être humain.

Certains acides de formule V, de même que des dérivés de ces acides, sont des agents neurotropes et psychotropes biens connus, ces propriétés ayant été publiées dans les brevets cités ci-dessus.

En outre, on a trouvé que des esters de l'invention sont supérieurs au reste médicamenteux correspondant de formule V du point de vue de leur toxicité.

Les esters de formule I, qui se sont révélés particulièrement intéressants dans le traitement des troubles neurologiques centraux, en particulier l'épilepsie, sont ceux dans lesquels le radical acyle représente un radical di-n-propylacétyle. Ces esters sont utiles en vue de provoquer une libération lente d'acide di-n-propylacétique.

Cet acide, connu également sous le nom d'acide valproïque, ainsi que certains de ses sels, constitue l'un des meilleurs agents antiépileptiques connus à l'heure actuelle : il est d'ailleurs largement commercialisé dans cette indication.

Chez l'être humain, le bis-(di-n-propylacétate)-1,2 de glycéryle et le bis-(di-n-propylacétate)-1,3 de glycéryle présentent un intérêt tout particulier, de même que les mélanges de ces diesters de glycéryle, ainsi qu'il sera discuté en détail par la suite.

Des études pharmacocinétiques entreprises avec des mono- et di-esters de l'invention dans lesquels le radical acyle représente un radical di-n-propylacétyle ont montré que ces composés présentent des différences tout à fait surprenantes comparativement au tri-(di-n-propylacétate) de glycéryle et au bis-(di-n-propylacétate) de propanediol-1,2.

Le tri-(di-n-propylacétate) de glycéryle est un composé connu ayant été publié par MEIJER et MEINARDI dans « Clinical and Pharmacological aspects of sodium valproate (Epilim) in the treatment of epilepsy » pp. 71-74 (compte rendu d'un Symposium tenu à l'Université de Nottingham, 23/24 septembre 1975).

Cet ester fut présenté comme « une substance remarquablement utile possédant toutes les propriétés attendues d'une composition à libération lente ».

De même, J.W.A. MEIJER et R. KALFF conclurent au Symposium de BETHEL (1974 — République Fédérale d'Allemagne — « Antiepileptic drugs » p. 222 à 228 édité par Schneider-Heidelberg), que le tri-(di-n-propylacétate) de glycéryle peut se comporter comme une forme à libération lente de l'acide di-n-propylacétique. Ils montrèrent que le taux sanguin maximum en acide di-n-propylacétique obtenu après administration chez l'être humain d'une dose orale de tri-(di-n-propylacétate) de glycéryle équivalent à 600 mg d'acide di-n-propylacétique est d'environ 30 µg/ml, 8 heures après administration.

Cependant, le tri-(di-n-propylacétate) de glycéryle ne peut constituer un agent thérapeutique valable car les taux sanguins en acide di-n-propylacétique qu'il permet d'atteindre varient de 10 à 20 µg/ml, ces taux étant trop faibles pour constituer des taux sanguins thérapeutiques. Des essais pratiqués dans le cadre de la présente invention ont corroboré ces résultats chez l'animal.

Ces essais effectués chez des rats ayant reçu 600 mg/kg de tri-(di-n-propylacétate) de glycéryle, par voie orale, montrèrent que l'acide di-n-propylacétique n'apparaît dans le sang que 150 minutes après administration et que les taux sanguins maximum en cet acide n'excèdent par 10 µg/ml.

La faible biodisponibilité de ce triglycéride est probablement due à un mauvais métabolisme de ce produit en glycérol et en acide di-n-propylacétique.

Au vu de ces résultats, il apparaît évident que le tri-(di-n-propylacétate) de glycéryle n'a aucune valeur comme produit permettant une libération lente de l'acide di-n-propylacétique bien que ce triester ait été présenté comme tel dans la littérature.

Le bis-(di-n-propylacétate) de propanediol-1,2, quant à lui, est un composé couvert de manière générale par le susdit brevet N° 1.289.620 de la République Fédérale d'Allemagne.

Des essais effectués chez l'homme, dans le cadre de la présente invention, ont révélé que l'administration orale de ce diester permet une libération lente de l'acide di-n-propylacétique.

Cependant, les taux sanguins en cet acide ainsi produit n'atteignent pas de seuils thérapeutiques.

Des essais pharmacocinétiques effectués chez l'animal, ont révélé que le (di-n-propylacétate)-1 de glycéryle, le bis-(di-n-propylacétate)-1,2 de glycéryle et le bis-(di-n-propylacétate)-1,3 de glycéryle possèdent les qualités requises pour provoquer la libération de l'acide di-n-propylacétique à vitesse décroissante dans l'organisme animal. Cependant, on a trouvé, par la suite, de manière tout à fait surprenante et imprévisible, que parmi les esters formés à partir d'acide di-n-propylacétique et du glycérol ou du propanediol seuls les diesters de glycérol à savoir le bis-(di-n-propylacétate)-1,2 de glycéryle et le bis-(di-n-propylacétate)-1,3 de glycéryle, sont capables de provoquer une libération lente de l'acide di-n-propylacétique chez l'être humain à un degré permettant d'atteindre des taux sanguins thérapeutiques en cet acide.

Des tests de pharmacocinétique pratiqués dans le cadre de l'invention ont montré que les taux sanguins en acide di-n-propylacétique obtenus après administration orale de 950 mg de tri-(di-n-propylacétate) de glycéryle chez l'homme sont trop faibles et, dans tous les cas, inférieurs à 20 µg/ml au taux sanguin maximum atteint alors que les taux sanguins thérapeutiques en cet acide sont compris entre 50 et 100 µg/ml c'est-à-dire entre environ 300 et 600 µmoles/ml.

Cette conclusion est en accord avec les résultats trouvés par J.W.A. MEIJER et R. KALFF cités précédemment.

La faible biodisponibilité de ce triglycéride est probablement due à un mauvais métabolisme de ce composé comme dans le cas de l'animal. Quant au (di-n-propylacétate)-1 de glycéryle, le métabolisme en glycérol et en acide di-n-propylacétique fut trouvé trop rapide. De même, les taux sanguins en acide di-n-propylacétique produits chez l'homme par l'administration orale de 1 000 mg de bis-(di-n-propylacétate) de propanediol-1,2 atteignent un maximum inférieur à 15 µg/ml (100 mmole/l) alors que 1 000 mg de mélange de bis-(di-n-propylacétate)-1,3 de glycéryle/bis-(di-n-propylacétate)-1,2 de glycéryle (« Mélange B ») produit dans les mêmes conditions des taux sanguins élevés en acide di-n-propylacétique. Ces taux sanguins sont environ 4 fois plus élevés (supérieurs à 400 m mole/l) que ceux obtenus au départ du diester de propanediol atteignant de ce fait des seuils thérapeutiques. On trouvera, par la suite, les résultats de quelques tests pharmacologiques et de pharmacocinétique montrant les qualités particulières des composés de la présente invention :

IV. Etude pharmacologique

On a cité ci-dessous les résultats d'essais effectués avec des dérivés di-n-propyl acétyles de l'invention en vue de déterminer l'activité anticonvulsivante.

A. Crise pentylènetétrazolique

L'activité anticonvulsivante a été prouvée en utilisant le pentylènetétrazol comme agent convulsivant.

Des lots de 10 rats ont été traités par voie orale, avec une dose de 600 mg/kg de (di-n-propylacétate)-1 de glycéryle.

A différents temps après l'administration, les animaux ont reçu une dose intrapéritonéale de 125 mg/kg de pentylènetétrazol.

On a alors déterminé le pourcentage de protection contre la crise convulsive aux différents temps en question.

Un essai parallèle a également été effectué avec 100 mg/kg d'acide di-n-propylacétique.

Cette dose représente la quantité d'acide di-n-propylacétique nécessaire pour atteindre un taux sanguin en acide di-n-propylacétique équivalent au taux maximum obtenu avec 600 mg/kg de (di-n-propylacétate)-1 de glycéryle.

Les résultats obtenus sont cités dans le Tableau suivant dans lequel on a renseigné le pourcentage de protection atteint aux différents temps considérés :

| Composé | Temps en heures après l'injection de pentylènetétrazol | | | | | |
|---|---|---|---|---|---|---|
| | 0,5 | 1 | 2 | 4 | 8 | 16 |
| (Di-n-propylacétate)-1 de glycéryle | 29 | 56 | 41 | 43 | 35 | 22 |
| Acide di-n-propylacétique | 23 | 0 | 0 | 0 | 0 | 0 |

B. Epilepsie chronique au cobalt

Cet essai a été effectué en comparaison au di-n-propylacétate de sodium. Des lots de rats ont reçu, pendant 14 jours, soit deux doses journalières intrapéritonéales de 200 mg/kg (matin et soir) de di-n-propylacétate de sodium soit une dose journalière intrapéritonéale de 400 mg/kg (soir) d'un mélange de bis-(di-n-propylacétates)-1,2 et -1,3 de glycéryle (« Mélange A »).

Des tracés encéphalographiques ont été relevés chaque matin en comparaison avec des animaux témoins. Les résultats sont donnés ci-dessous :

(Voir tableau, p. 11)

| | Témoins | Di-n-propylacétate de sodium | Mélange de diglycérides |
|---|---|---|---|
| Nombre de rats présentant au moins une crise | 7/7 | 3/4 | 0/2 |
| Nombre de crises entre le 7ème et le 14ème jour | 3,57 | 1,25 | 0 |

V. Etude pharmacocinétique

Les résultats obtenus lors d'études pharmacocinétiques effectuées avec des esters de l'invention ressortiront de la description qui va suivre en référence aux dessins annexés.

A. Etude pharmacocinétique chez l'animal

La première série de figures représente des taux sanguins en acide de formule V ci-dessus par rapport aux temps, cet acide étant libéré soit à partir d'un de ses sels soit à partir d'un ester de l'invention.

Figure 1 représente des taux sanguins en acide di-n-propylacétique obtenus à partir :

a) D'une dose orale, chez le rat, de 600 mg/kg :
de (di-n-propylacétate)-1 de glycéryle (Courbe 1)
ou de bis-(di-n-propylacétate)-1,2 de glycéryle (Courbe 2)
ou de bis-(di-n-propylacétate)-1,3 de glycéryle (Courbe 3)
b) D'une dose intraveineuse, chez le rat, de 200 mg/kg de di-n-propylacétate de sodium (Courbe 4)

Les courbes montrent que, chez l'animal, le (di-n-propylacétate)-1 de glycéryle, lorsqu'il est métabolisé en acide di-n-propylacétique, s'oppose à l'élimination trop rapide de cet acide et maintient un taux élevé en cet acide.

En outre, le comportement pharmacocinétique de ces deux diesters est très similaire. Les taux sanguins en acide di-n-propylacétique obtenus à partir de ces esters sont très semblables à ceux observés de la 2ᵉ à la 24ᵉ heure après l'injection de di-n-propylacétate de sodium.

Figure 2 repésente des taux sanguins en acide méthyl-2 éthyl-2 hexanoïque obtenus à partir :

a) D'une dose orale, chez le lapin, de :
596 mg/kg de (méthyl-2 éthyl-2 hexanoate)-1 de glycéryle (Courbe 1)
207 mg/kg de bis-(méthyl-2 éthyl-2 hexanoate)-1,2 de glycéryle (Courbe 2)
b) D'une dose intraveineuse chez le lapin, de 82 mg/kg de méthyl-2 éthyl-2 hexanoate de sodium (Courbe 3)

Figure 3 représente des taux sanguins en acide méthyl-1 cyclohexylcarboxylique obtenus à partir :

a) D'une dose orale, chez le rat, de 600 mg/kg :
de (méthyl-1 cyclohexylcarboxylate)-1 de glycéryle (Courbe 1)
ou de bis-(méthyl-1 cyclohexylcarboxylate)-1,2 de glycéryle (Courbe 2)
ou de tri-(méthyl-1 cyclohexylcarboxylate)-1,2,3 de glycéryle (Courbe 3)
b) D'une dose intraveineuse, chez le rat, de 200 mg/kg de méthyl-1 cyclohexylcarboxylate de sodium (Courbe 4)

Les résultats montrent que le monoglycéride permet d'obtenir des taux sanguins en acide méthyl-1 cyclohexylcarboxylique plus élevés que le diglycéride, ce dernier étant lui-même mieux métabolisé en acide méthyl-1 cyclohexylcarboxylique que le triglycéride.

Figure 4 représente des taux sanguins en acide di-n-propylacétique obtenus à partir :

a) D'une dose orale, chez le lapin, de 562 mg/kg de (di-n-propylacétate)-1 (méthyl-1 cyclohexylcarboxylate)-2 de glycéryle (Courbe 1)
b) D'une dose intraveineuse, chez le lapin, de 200 mg/kg de di-n-propylacétate de sodium (Courbe 2)

Figure 5 représente des taux sanguins en acide méthyl-1 cyclohexylcarboxylique obtenus à partir :

a) D'une dose orale, chez le lapin, de 562 mg/kg de (di-n-propylacétate)-1 (méthyl-1 cyclohexylcarboxylate)-2 de glycéryle (Courbe 1)
b) D'une dose intraveineuse, chez le lapin, de 100 mg/kg de méthyl-1 cyclohexylcarboxylate de

sodium (Courbe 2).

Les résultats montrent que des taux sanguins stables en acide di-n-propylacétique et en acide méthyl-1 cyclohexylcarboxylique peuvent être maintenus pendant au moins 30 heures avec le diglycéride.

B. Etude pharmacocinétique chez l'être humain

Figure 6 représente des taux sanguins en acide di-n-propylacétique obtenus à partir :

a) D'une dose orale de 600 mg de (di-n-propylacétate)-1 de glycéryle (Courbe 1)
b) D'une dose orale de 476 mg de bis-(di-n-propylacétate)-1,2 de glycéryle (Courbe 2)
c) D'une dose orale de 460 mg de di-n-propylacétate de sodium (Courbe 3)

Les résultats montrent que les taux sanguins en acide di-n-propylacétique produits par la métabolisation du (di-n-propylacétate)-1 de glycéryle sont très voisins des taux sanguins obtenus après administration de di-n-propylacétate de sodium (460 mg de di-n-propylacétate de sodium équivalent à 600 mg de (di-n-propylacétate)-1 de glycéryle en ce qui concerne la quantité d'anion di-n-propylacétyle).

Par conséquent, l'administration orale de 600 mg de (di-n-propylacétate)-1 de glycéryle ne présente aucun avantage comparativement à 460 mg de di-n-propylacétate de sodium.

Ce résultat prouve que le monoester en question est tout à fait inadéquat pour induire une libération lente d'acide di-n-propylacétique dans le sang.

Figure 7 représente des taux sanguins en acide di-n-propylacétique obtenus à partir de l'administration orale de 1 000 mg de bis-(di-n-propylacétate)-1,2 de glycéryle chaque 24 heures pendant 4 jours.

Les résultats montrent que les taux sanguins en acide di-n-propylacétique varient d'environ 300 à 550 µmoles/1 (50 à 90 µg/ml).

Par conséquent, une seule administration orale de ce diester-1,2 chaque 24 heures est capable de maintenir des taux thérapeutiques en acide di-n-propylacétique.

Ce niveau ne peut être atteint avec trois administrations orales journalières chacune de 400 mg de di-n-propylacétate de sodium.

Des résultats très semblables ont été enregistrés chez l'être humain avec le bis-(di-n-propylacétate)-1,3 de glycéryle.

Ce composé semble même présenter une meilleure biodisponibilité que celle du diester-1,2 correspondant.

Figure 8 représente des taux sanguins en acide di-n-propylacétique obtenus à partir :

a) D'une dose orale de 1 000 mg de mélanges de bis-(di-n-propylacétate)-1,2 de glycéryle/bis-(di-n-propylacétate)-1,3 de glycéryle (« Mélanges A et B » cités précédemment) (Courbes 1 à 5)
b) D'une dose orale de 1 000 mg de di-n-propylacétate de sodium (Courbe 6).

Les résultats montrent que la cinétique de libération de l'acide di-n-propylacétique à partir des mélanges précités est très semblable à celle obtenue avec le diester-1,2 seul ou le diester-1,3 seul.

La demi-vie apparente de l'acide di-n-propylacétique ainsi libéré à partir de ces mélanges est de 20 h 20 min. alors que celle de l'acide di-n-propylacétique après l'administration orale du sel de sodium de ce composé est de 11 h 35 min.

De l'ensemble des résultats des essais précités, on peut conclure que le bis-(di-n-propylacétate)-1,2 de glycéryle, le bis-(di-n-propylacétate)-1,3 de glycéryle, et les mélanges de diesters di-n-propylacétiques du glycérol tels que définis précédemment, présentent, chez l'être humain, des avantages importants qui peuvent se résumer comme suit :

— les taux sanguins élevés en acide di-n-propylacétique apparaissant 30 min. à 1 heure après l'administration orale de 300 à 500 mg de di-n-propylacétate de sodium peuvent être évités, ces taux provoquant des effets secondaires indésirables tels que des vertiges.

— la posologie est rendue plus aisée puisqu'une seule administration journalière, à savoir environ 500 mg/25 kg de poids corporel/24 heures, donne naissance, à partir du quatrième jour après la première administration, à des taux sanguins thérapeutiques constants variant entre 50 et 100 µg/ml en acide di-n-propylacétique. Cet avantage ne peut être obtenu avec plusieurs administrations journalières de di-n-propylacétate de sodium actuellement disponible sur le marché même lorsque ce produit est donné sous une forme galénique connue à libération lente

— la protection thérapeutique est meilleure parce que le taux sanguin en acide di-n-propylacétique est plus stable.

En outre, on a pu démontrer que les composés de l'invention sont extrêmement peu toxiques.

On trouvera, ci-après, les résultats de tests de toxicité aiguë obtenus avec des composés de l'invention :

a) Bis-(di-n-propylacétate)-1,2 de glycéryle

$DL_0$ chez le rat par voie orale : > 5 000 mg/kg

$DL_0$ chez le rat par voie intrapéritonéale : > 2 000 mg/kg

Des résultats semblables ont été obtenus chez la souris.

b) (Di-n-propylacétate)-1 de glycéryle
$DL_{50}$ chez le rat par voie orale : 2 365 mg/kg
$DL_{50}$ chez la souris par voie orale : 3 361 mg/kg

c) Mélange de bis-(di-n-propylacétate)-1,2 de glycéryle/bis-(di-n-propylacétate)-1,3 de glycéryle (« Mélange A »)

$DL_0$ chez le rat par voie orale : > 5 000 mg/kg
$DL_{50}$ chez le rat par voie intrapéritonéale : 2 368 mg/kg
$DL_0$ chez la souris par voie orale : > 5 000 mg/kg
$DL_{50}$ chez la souris par voie intrapéritonéale : 2 515 mg/kg

d) Mélange de bis-(di-n-propylacétate)-1,2 de glycéryle/bis-(di-n-propylacétate)-1,3 de glycéryle (« Mélange B »)

$DL_0$ chez le rat par voie orale : > 5 000 mg/kg
$DL_{50}$ chez le rat par voie intrapéritonéale : 2 368 mg/kg
$DL_{50}$ chez la souris par voie orale : 5 597 mg/kg
$DL_{50}$ chez la souris par voie intrapéritonéale : 1 920 mg/kg

En comparaison, la $DL_{50}$ du di-n-propylacétate de sodium par voie orale chez le rat et la souris est respectivement de 1 530 mg/kg et 1 700 mg/kg.

Les compositions thérapeutiques selon l'invention peuvent être présentées sous toute forme convenant à l'administration en thérapie humaine ou vétérinaire. Pour ce qui concerne l'unité d'administration, celle-ci peut prendre forme, par exemple, d'un comprimé, d'une dragée, d'une microcapsule, d'une gélule, d'une émulsion aqueuse ou d'une ampoule buvable pour l'administration orale ou d'une solution ou suspension pour l'administration parentérale.

L'unité d'administration pourra comprendre par exemple de 50 à 600 mg de principe actif pour l'administration orale.

Suivant la voie d'administration choisie, les compositions pharmaceutiques ou vétérinaires de l'invention seront préparées en associant au moins un des composés de l'invention avec au moins un excipient ou véhicule approprié, ce dernier pouvant être constitué d'au moins un ingrédient sélectionné parmi les substances suivantes : gélatine, glycérol, p-hydroxybenzoate d'éthyle (sodé), p-hydroxybenzoate de propyle (sodé).

Les Exemples suivants illustrent la préparation des esters de glycérol de l'invention de même qu'une composition thérapeutique les contenant :

## Exemple 1

Préparation du (di-n-propylacétate-1) de glycéryle

A. (Di-n-propylacétyl)-1 isopropylidène-2,3 glycérol ou diméthyl-2,2 (di-n-propylacétoxyméthyl)-4 dioxolanne-1,3

a) Isopropylidène-1,2 glycérol

Dans un ballon de 1 000 ml, on introduit 92,1 g (1 mole) de glycérol, 217 g (3,75 moles) d'acétone, 179 g (275 ml) d'éther de pétrole et 2,75 g (0,015 mole) d'acide p-toluènesulfonique. Sous agitation, on chauffe le mélange sous reflux et on élimine l'eau ainsi formée jusqu'à fin de décantation.

Cette opération est accomplie en 25 à 30 heures. Après refroidissement, on neutralise l'acide p-toluènesulfonique en ajoutant 1,20 g (0,009 mole) de carbonate de potassium. Trente minutes plus tard, on filtre le milieu sous agitation. On transfère le filtrat dans un ballon de 1 000 ml équipé d'un réfrigérant et on élimine les solvants sous pression atmosphérique et à la température de 90 °C dans la masse.

On distille le concentrat sous pression réduite et on recueille la fraction bouillant à 86 °C ± 1° sous 12 mm Hg.

De cette manière, on obtient 95 g d'isopropylidène-1,2 glycérol sous forme d'un liquide incolore.
Rendement : 72 %

En utilisant la même méthode que celle décrite ci-dessus, on a préparé les composés suivants :

Composés

Méthyl-2 éthyl-2 hydroxyméthyl-4 dioxolane-1,3
Rendement : 54,7 %
$n_D^{20} = 1,440\,4$

Spectre infra-rouge (film) : OH large à 3 450 $cm^{-1}$

$CH_3$, $CH_2$ à 2 990 $cm^{-1}$ (F)
2 950 $cm^{-1}$ (F)
2 890 $cm^{-1}$ (F)
C—O—C large à 1 060 $cm^{-1}$

Méthyl-2 isobutyl-2 hydroxyméthyl-1,3 dioxolanne
Rendement : 75,2 %
$n_D^{20}$ = 1,443 2

Spectre infra-rouge (film) : OH large à 3 450 $cm^{-1}$
$CH_3$, $CH_2$, CH à 3 000 $cm^{-1}$
2 970 $cm^{-1}$ (F)
2 940 $cm^{-1}$ (F)
2 880 $cm^{-1}$ (F)
C—O—C à 1 045 $cm^{-1}$

Cyclohexylidène-1,2 glycérol
Rendement : 35 %
$n_D^{20}$ = 1,480 1

Spectre infra-rouge (film) : OH large à 3 450 $cm^{-1}$
$CH_3$, $CH_2$ à 2 950 $cm^{-1}$ (F)
2 870 $cm^{-1}$ (m)
C—O—C à 1 110 $cm^{-1}$ (F)

b) (Di-n-propylacétyl)-1 isopropylidène-2,3 glycérol

Dans un ballon parfaitement sec, on introduit 132,2 g (1 mole) d'isopropylidène-1,2 glycérol, 118,7 g (1,5 mole) de pyridine et 271 g (290 ml) de N,N-diméthylformamide.

Sous agitation, on introduit 162,7 g (1 mole) de chlorure de di-n-propylacétyle. Cette opération nécessite 30 minutes. Le milieu réactionnel est alors chauffé à 65 °C ± 5 °C pendant 2 heures, refroidi à température ambiante et hydrolysé avec 1 200 g d'eau distillée. Après cette opération, on extrait sous agitation pendant 30 minutes avec deux fractions chacune de 700 g (528 ml) de chlorure de méthylène. On recueille les fractions organiques et on les lave successivement avec 300 g d'une solution aqueuse à 5 % de bicarbonate de sodium et trois fractions chacune de 300 g d'eau distillée. Après décantation, on sèche le mélange sur sulfate de sodium, on filtre et on rince avec une quantité minimum de chlorure de méthylène. On élimine le solvant sous pression réduite à la température de 50 °C maximum. On distille le concentrat sous pression réduite et on recueille la fraction bouillant à 93 °C ± 2° sous 1 mm Hg.

De cette manière, on obtient 224,5 g de (di-n-propylacétyl)-1 isopropylidène-2,3 glycérol sous la forme d'un liquide incolore.

Rendement : 87 %
$n_D^{21}$ = 1,434 4
Spectre infra-rouge : $CH_3$, $CH_2$ à 2 970 $cm^{-1}$ (F)
2 940 $cm^{-1}$ (F)
2 880 $cm^{-1}$ (m)
CO ester à 1 740 $cm^{-1}$ (F)
1 175 $cm^{-1}$ (m)
C—O—C à 1 060 $cm^{-1}$

En utilisant la même méthode que précédemment, on a préparé les composés suivants :

Composés

(Di-n-propylacétyl)-1 cyclohexylidène-2,3 glycérol
Rendement : 74,7 %
$n_D^{21}$ = 1,458 3
Spectre infra-rouge (film) : $CH_3$, $CH_2$ à 2 950 $cm^{-1}$ (F)
2 875 $cm^{-1}$ (F)
CO ester à 1 740 $cm^{-1}$ (F)
1 770 $cm^{-1}$ (F)
C—O—C à 1 100 $cm^{-1}$ (F)

(Tri-n-propylacétyl)-1 isopropylidène-2,3 glycérol (après sodation de l'isopropylidène-2,3 glycérol en excès et estérification à 88/90 °C dans le N,N-diméthylformamide)
Rendement : 88,7 %
$n_D^{22}$ = 1,443 0
Spectre infra-rouge (film) : $CH_3$, $CH_2$ à 2 970 $cm^{-1}$ (F)
2 945 $cm^{-1}$ (m)

2 880 $cm^{-1}$ (F)
CO ester à 1 735 $cm^{-1}$ (F)
1 210 $cm^{-1}$ (F)
C—O—C à 1 145 $cm^{-1}$ (F)

(Ethyl-2 hexanoyl)-1 isopropylidène-2,3 glycérol
Rendement : 77,5 %
$n_D^{20}$ = 1,434 1
Spectre infra-rouge (film) : $CH_3$, $CH_2$ à 2 980 $cm^{-1}$ (F)
2 950 $cm^{-1}$ (F)
2 990 $cm^{-1}$ (m)
CO ester à 1 745 $cm^{-1}$ (F)
1 175 $cm^{-1}$ (F)
C—O—C à 1 220 $cm^{-1}$ (F)

(Méthyl-1 cyclohexylcarbonyl)-1 isopropylidène-2,3 glycérol
Rendement : 67,3 %
$n_D^{20}$ = 1,456 2
Spectre infra-rouge (film) : $CH_3$, $CH_2$ à 3 000 $cm^{-1}$ (m)
2 950 $cm^{-1}$ (F)
2 865 $cm^{-1}$ (m)
CO ester à 1 735 $cm^{-1}$ (F)
1 165 $cm^{-1}$ (m)

(Méthyl-2 éthyl-2 hexanoyl)-1 isopropylidène-2,3 glycérol (sodation de l'isopropylidène-1,2 glycérol)
Rendement : 74,3 %
Spectre infra-rouge (film) : $CH_3$, $CH_2$ à 2 970 $cm^{-1}$ (F)
2 895 $cm^{-1}$ (F)
CO ester à 1 735 $cm^{-1}$ (F)
1 160 $cm^{-1}$ (F)

(Di-n-propylméthoxyacétyl)-1 isopropylidène-2,3 glycérol
(n-Propyl-4 heptanoyl)-1 isopropylidène-2,3 glycérol
(n-Propyl-5 octanoyl)-1 isopropylidène-2,3 glycérol

B. (Di-n-propylacétate)-1 de glycéryle

Dans un ballon, on introduit 258,4 g (1 mole) de (di-n-propylacétyl)-1 isopropylidène-2,3 glycérol, 410 g (520 ml) de méthanol et 125 ml d'acide chlorhydrique 3N. On maintient le milieu réactionnel sous agitation à température ambiante pendant environ 3 heures. Après cette opération, on élimine les solvants sans dépasser 40 °C dans la masse. On transfère le concentrat dans une ampoule à décanter et on extrait avec deux fractions chacune de 860 g (650 ml) de chlorure de méthylène. On recueille les extraits et on les lave successivement avec 2 fractions chacune de 390 ml d'une solution aqueuse à 5 % de bicarbonate de sodium et 2 fractions chacune de 400 ml d'eau distillée. Après séchage sur sulfate de sodium, on filtre le mélange. On évapore le solvant à une température inférieure à 50 °C puis sous vide d'environ 1 mm Hg jusqu'à poids constant.

De cette manière, on obtient 203 g de (di-n-propylacétate)-1 de glycéryle sous forme d'un liquide pratiquement incolore.
$n_D^{20}$ = 1,451 3
Spectre infra-rouge (film) : OH large à 3 470 $cm^{-1}$
$CH_3$, $CH_2$ à 2 970 $cm^{-1}$ (F)
2 950 $cm^{-1}$ (F)
2 890 $cm^{-1}$ (F)
CO ester à 1 740 $cm^{-1}$ (F)
épaulement à 1 720 $cm^{-1}$
1 180 $cm^{-1}$ (m)

En utilisant la même méthode que celle décrite précédemment, on a préparé les composés suivants :

Composés

(Tri-n-propylacétate)-1 de glycéryle
Rendement : 92 %
$n_D^{20}$ = 1,456 1
Spectre infra-rouge (film) : OH large à 3 430 $cm^{-1}$
$CH_3$, $CH_2$ à 2 965 $cm^{-1}$ (F)
CH à 2 880 $cm^{-1}$ (F)
CO ester à 1 730 $cm^{-1}$ (F)
1 215 $cm^{-1}$ (F)

(Ethyl-2 hexanoate)-1 de glycéryle
Rendement : 95 %

$n_D^{22}$ = 1,451 1
Spectre infra-rouge (film) : OH large à 3 530 $cm^{-1}$
$CH_3$, $CH_2$ à 2 970 $cm^{-1}$
2 945 $cm^{-1}$
2 880 $cm^{-1}$
CO ester à 1 740 $cm^{-1}$ (F)
épaulement à 1 725 $cm^{-1}$
1 180 $cm^{-1}$ (m)

(Méthyl-1 cyclohexylcarboxylate)-1 de glycéryle
Rendement : 87 %
$n_D^{20}$ = 1,476 9
Spectre infra-rouge (film) : OH large à 3 420 $cm^{-1}$
$CH_3$, $CH_2$ à 2 940 $cm^{-1}$ (F)
2 865 $cm^{-1}$ (F)
CO ester à 1 725 $cm^{-1}$ (F)
1 170 $cm^{-1}$ (F)

(Méthyl-2 éthyl-2 hexanoate)-1 de glycéryle
Rendement : 92 %
$n_D^{22,5}$ = 1,453 2
Spectre infra-rouge (film) : OH large à 3 420 $cm^{-1}$
$CH_3$, $CH_2$ à 2 970 $cm^{-1}$ (F)
2 940 $cm^{-1}$ (F)
2 880 $cm^{-1}$ (m)
CO ester à 1 730 $cm^{-1}$ (F)

(Di-n-propylméthoxyacétate)-1 de glycéryle
Rendement : 40,5 %
$n_D^{14}$ = 1,459 3
Spectre infra-rouge (film) : OH à 3 400 $cm^{-1}$ (F)
C = O à 1 755 $cm^{-1}$ (F)
CO ester à 1 200 $cm^{-1}$ (F)

(n-Propyl-4 heptanoate)-1 de glycéryle
Rendement : 42,6 %
$n_D^{20}$ = 1,458 5
Spectre infra-rouge (film) : OH à 3 400 $cm^{-1}$ (m)
C = O à 1 740 $cm^{-1}$ (F)
CO ester à 1 175 $cm^{-1}$ (m)

(n-Propyl-5 octanoate)-1 de glycéryle
Rendement : 67,5 %
$n_D^{20}$ = 1,459 5
Spectre infra-rouge (film) : OH à 3 400 $cm^{-1}$ (F)
C = O à 1 740 $cm^{-1}$ (F)
CO ester à 1 170 $cm^{-1}$ (m)

## Exemple 2

Préparation du (di-n-propylacétate)-2 de glycéryle

a) Benzylidène-1,3 glycérol ou phényl-2 dioxanne-1,3 ol-5

Dans un ballon de 500 ml, on introduit 92,1 g (1 mole) de glycérol, 78 g (100 ml) de benzène, 111,3 g (1,05 mole) de benzaldéhyde et 0,2 g d'acide p-toluènesulfonique.

Sous agitation, on chauffe le mélange au reflux et on élimine l'eau ainsi formée. Cette opération nécessite environ 4 heures. On évapore le solvant sous vide et on obtient ainsi un résidu huileux d'environ 188 g. On distille ce résidu et on recueille la fraction bouillant à 114 ± 2 °C sous 0,1 mm Hg. Après quoi, on fait barboter 0,35 g d'acide chlorhydrique gazeux dans le distillat, on homogénéise le milieu et on abandonne pendant 8 jours au réfrigérateur.

Après 12 heures à 0 °C, une masse se forme. On dissout cette masse par chauffage, dans environ 1 075 g (1 500 ml) d'éther éthylique. On lave la phase organique avec 70 ml d'ammoniaque (1 % en $NH_3$), sèche sur sulfate de sodium et cristallise à − 5 °C.

Après essorage, on sèche le produit sous vide à 40 °C jusqu'à poids constant.

De cette manière, on obtient environ 80 g de benzylidène-1,3 glycérol.

Rendement : environ 45 %

b) Benzylidène-1,3 (di-n-propylacétyl)-2 glycérol

Dans un ballon, on introduit 180,2 g (1 mole) de benzylidène-1,3 glycérol brut et 782,5 g (1,5 mole) de pyridine anhydre.

Sous agitation, on ajoute 244 g de chlorure de di-n-propylacétyle à température ambiante. Cette opération nécessite 20 minutes. On chauffe le mélange à 60 °C et on l'y maintient pendant 2 heures. On élimine la pyridine en excès à 50 °C sous vide et on reprend le résidu dans 715 g (1 000 ml) d'éther éthylique. On extrait le milieu avec une solution aqueuse à 5 % de carbonate de potassium pour obtenir un pH = 7 à 8 puis avec de l'eau distillée pour obtenir un pH = 6 à 7. Après séchage sur sulfate de sodium, on filtre le mélange, on évapore le solvant sous vide et on rectifie par distillation l'extrait ainsi obtenu. On recueille la fraction bouillant entre 150 et 152 °C sous 0,5 mm Hg.

De cette manière, on obtient 257 g de benzylidène-1,3 (di-n-propylacétyl)-2 glycérol.
Rendement : 83,9 %

c) (Di-propylacétate)-2 de glycéryle

Dans un ballon de 2 000 ml, on introduit 947 g (1 200 ml) d'éthanol absolu, 122,6 g (0,4 mole) de benzylidène-1,3 (di-n-propylacétyl)-2 glycérol et 8 g de charbon palladié à 5 %. On purge alors l'appareil avec de l'azote. Après quoi, on y fait barboter 18,8 l d'hydrogène à température ambiante et sous pression atmosphérique. Cette opération nécessite 7 heures. On purge alors l'appareil avec l'azote et on filtre le catalyseur. On chauffe le filtrat à 50 °C maximum et jusqu'à poids constant sous 1 mm Hg.

De cette manière, on obtient, 87,7 g de (di-n-propylacétate)-2 de glycéryle sous la forme d'un liquide incolore légèrement visqueux.

Rendement : quantitatif
$n_D^{22}$ = 1,451 2
Spectre infra-rouge (film) : OH large à 3 450 $cm^{-1}$
$CH_3$, $CH_2$ à 2 970 $cm^{-1}$ (F)
2 950 $cm^{-1}$ (F)
2 890 $cm^{-1}$ (m)
CO ester à 1 745 $cm^{-1}$ (F)
1 720 $cm^{-1}$ (F)
C—O à 1 180 $cm^{-1}$ (m)

Exemple 3

Préparation du bis-(di-n-propylacétate)-1,2 de glycéryle

a) Benzyl-1 isopropylidène-2,3 glycérol

Dans un ballon parfaitement sec de 2 000 ml, on introduit 500 g (575 ml) de toluène et 19,8 g (0,86 at.g) de sodium.

On chauffe le mélange au reflux et sous vive agitation pour obtenir une bonne dispersion du sodium. Après cette opération, on refroidit le milieu toujours sous vive agitation.

A la température de + 10/+ 20 °C, on ajoute alors lentement 132,2 g (1 mole) d'isopropylidène-1,2 glycérol, ce qui nécessite environ 30 minutes. On chauffe alors le milieu réactionnel au reflux pendant environ 1 heure puis, après refroidissement à la température ambiante, on ajoute, en une fois, 108,9 g de chlorure de benzyle. On chauffe progressivement au reflux pendant environ 1 heure et on maintient sous reflux total pendant 30 minutes. Après refroidissement, on évapore le toluène sous pression réduite et on ajoute 1 070 g (1 500 ml) d'éther éthylique. On filtre les sels et on les rince avec 360 g (500 ml) d'éther éthylique. On élimine le solvant sous pression réduite et on rectifie le concentrat par distillation. On recueille alors la fraction bouillant à 125 °C ± 2° sous 3 mm Hg.

De cette manière, on obtient 166,7 g de benzyl-1 isopropylidène-2,3 glycérol.
Rendement : 75 %
P.E. : 117-121 °C sous 3 mm Hg.

b) Benzyl-1 glycérol

Dans un ballon, on introduit 222,3 g (1 mole) de benzyl-1 isopropylidène-2,3 glycérol et 567 g d'une solution aqueuse à 10 % d'acide acétique. Sous agitation, on chauffe le mélange au bain-marie (t° = 95 °C) pendant 1 heure. On élimine les solvants sous pression réduite et on rectifie le concentrat par distillation sous vide. On recueille alors la fraction bouillant entre 132-135 °C sous 0,6 mm Hg laquelle se présente sous forme d'un liquide incolore.

De cette manière, on obtient 160 g de benzyl-1 glycérol.
Rendement : 88 %
Spectre infra-rouge (film) : OH large à 3 400 $cm^{-1}$
C—H aromatique à 3 080 $cm^{-1}$ (f)
3 040 $cm^{-1}$ (m)

$CH_3$, $CH_2$ à 2 930 $cm^{-1}$ (F)
2 870 $cm^{-1}$ (F)
C = C aromatique à 1 610 $cm^{-1}$ (f)
1 590 $cm^{-1}$ (f)

c) Benzyl bis-(di-n-propylacétyl)-2,3 glycérol

Dans un ballon de 2 000 ml, on introduit 182,2 g (1 mole) de benzyl-1 glycérol et 978 g (1 000 ml) de pyridine anhydre.

Sous agitation à température ambiante, on ajoute lentement 341,2 g (2,1 moles) de chlorure de di-n-propylacétyle. On chauffe le milieu à 50-55 °C et on l'y maintient pendant 90 minutes. On élimine sous vide la pyridine en excès et on reprend le résidu dans 3 000 g d'eau distillée. Après extraction avec deux fractions chacune de 2 500 ml d'éther éthylique, on lave la phase organique successivement avec 3 000 ml d'une solution à 7 % d'acide chlorhydrique, 3 000 ml d'eau distillée, 3 000 ml d'une solution aqueuse à 10 % de bicarbonate de sodium et trois fractions chacune de 2 000 ml d'eau distillée. On sèche le mélange sur sulfate de sodium, on le filtre et on élimine le solvant sous vide. On purifie le produit brut ainsi obtenu par chromatographie sur colonne contenant 4 350 g de silice et en utilisant le toluène comme éluant.

De cette manière, on obtient 355,2 g de benzyl-1 bis-(di-n-propylacétyl)-2,3 glycérol.

Rendement : 81,7 %

En utilisant la même méthode que celle décrite précédemment, on a préparé les composés suivants :

Composés

Benzyl-1 bis-(éthyl-2 hexanoyl)-2,3 glycérol
Rendement : 72 %
Benzyl-1 bis-(méthyl-1 cyclohexyl carbonyl)-2,3 glycérol
Rendement : 70 %
Benzyl-1 bis-(tri-n-propylacétyl)-2,3 glycérol
Rendement : 51,7 %
Benzyl-1 bis-(éthyl-2 méthyl-2 hexanoyl)-2,3 glycérol
Rendement : 69,5 %

d) Bis-(di-n-propylacétate)-1,2 de glycéryle

Dans un appareil à hydrogénation, on introduit 4 350 ml d'éthanol pur, 434,3 g (1 mole) de benzyl-1 bis-(di-n-propylacétyl)-2,3 glycérol et 26 g de charbon palladié à 5 %. On purge l'appareil avec de l'azote et on y fait barboter de l'hydrogène pendant 9 heures à température ambiante et sous pression atmosphérique (22,4 l d'hydrogène à 0 °C/760 mm Hg). On purge à nouveau l'appareil avec de l'azote et on filtre le catalyseur.

On chauffe le filtrat à 50 °C et puis sous vide (1 mm Hg) jusqu'à siccité. De cette manière, on obtient 344 g de bis-(di-n-propylacétate)-1,2 de glycéryle sous la forme d'un liquide incolore.

Rendement : quantitatif
$n_D^{20} = 1,447$
Spectre infra-rouge (film) : OH large à 3 500 $cm^{-1}$
$CH_3$, $CH_2$ à 2 995 $cm^{-1}$ (F)
2 980 $cm^{-1}$ (F)
2 880 $cm^{-1}$ (F)
CO ester à 1 745 $cm^{-1}$ (F)
1 170 $cm^{-1}$ (F)

En utilisant le même procédé que précédemment, on a préparé les composés suivants :

Composés

Bis-(éthyl-2 hexanoate)-1,2 de glycéryle
Rendement : quantitatif
$n_D^{20} = 1,446\,8$
Spectre infra-rouge (film) : OH large à 3 500 $cm^{-1}$
$CH_3$, $CH_2$ à 2 975 $cm^{-1}$ (F)
2 950 $cm^{-1}$ (F)
2 890 $cm^{-1}$ (F)
CO ester à 1 745 $cm^{-1}$ (F)
épaulement à 1 725 $cm^{-1}$
1 175 $cm^{-1}$

Bis-(méthyl-1 cyclohexylcarboxylate)-1,2 de glycéryle
Rendement : quantitatif
$n_D^{20}$ = 1,477 3
Spectre infra-rouge (film) : OH large à 3 520 $cm^{-1}$
$CH_3$, $CH_2$ à 2 940 $cm^{-1}$ (F)
2 870 $cm^{-1}$ (F)
CO ester à 1 735 $cm^{-1}$ (F)
1 175 $cm^{-1}$ (F)

Bis-(tri-n-propylacétate)-1,2 de glycéryle
Rendement : quantitatif
$n_D^{20}$ = 1,456 1
Spectre infra-rouge (film) : OH large à 3 500 $cm^{-1}$
$CH_3$, $CH_2$ à 2 970 $cm^{-1}$ (F)
2 950 $cm^{-1}$ (F)
2 885 $cm^{-1}$ (F)
CO ester à 1 735 $cm^{-1}$ (F)
1 210 $cm^{-1}$ (m)

Bis-(méthyl-2 éthyl-2 hexanoate)-1,2 de glycéryle
Rendement : 78,7 %
$n_D^{22}$ = 1,451 9
Spectre infra-rouge (film) : OH large à 3 530 $cm^{-1}$
$CH_3$, $CH_2$ à 2 975 $cm^{-1}$ (F)
2 950 $cm^{-1}$ (F)
2 890 $cm^{-1}$ (F)
CO ester à 1 735 $cm^{-1}$ (F)
1 150 $cm^{-1}$ (F)

## Exemple 4

Préparation du bis-(di-n-propylacétate)-1,3 de glycéryle

a) Bis-(di-n-propylacétoxy)-1,3 acétone

Dans un ballon parfaitement sec, on introduit 680 g (935 ml) d'éther isopropylique, 173,7 g (2,2 moles) de pyridine anhydre et 90,1 g (1 mole) de dihydroxyacétone. Sous agitation à température ambiante, on ajoute 349,7 g (2,15 moles) de chlorure de di-n-propylacétyle en 30 minutes à 1 heure. On chauffe le milieu réactionnel au reflux à 65 °C et on l'y maintient pendant 3 heures. Après refroidissement, on hydrolyse le mélange avec 500 ml d'eau distillée glacée. On lave la couche organique avec une solution diluée d'acide chlorhydrique jusqu'à pH = 1, puis avec de l'eau jusqu'à neutralité. Après séchage sur sulfate de sodium, on évapore le solvant sous vide à 50 °C.

On rectifie le concentrat par distillation et on recueille la fraction bouillant entre 155-160 °C sous 0,2/0,3 mm Hg.

De cette manière, on obtient 205 g de bis-(di-n-propylacétoxy)-1,3 acétone brute.

Rendement : 60 %

b) Bis-(di-n-propylacétate)-1,3 de glycéryle

Dans un ballon, on introduit 342,48 g (1 mole) de bis-(di-n-propylacétoxy)-1,3 acétone, 2 490 g (2 800 ml) de tétrahydrofuranne et 59,3 g (1,1 mole) de borohydrure de potassium. Après quoi, on ajoute 880 g d'eau distillée et 110 g (1,1 mole) d'acide chlorhydrique à 36 % de façon à maintenir le pH du milieu entre 7 et 8.

Cette opération nécessite 1 heure. Si nécessaire, on ajuste le pH à 7-8. On abandonne le milieu pendant 1 heure puis on le chauffe jusqu'à siccité tout en maintenant la température inférieure à 45-50 °C. On reprend le résidu dans l'éther éthylique, on lave avec de l'eau pour éliminer les sels, on sèche sur sulfate de sodium et on chauffe jusqu'à siccité. On reprend le résidu dans le toluène et on le passe sur colonne contenant environ 3 000 g de silice. On élue le cétodiester résiduel avec du toluène et le produit désiré avec du chloroforme. On chauffe à siccité les fractions chloroformiques contenant le produit désiré à une température inférieure à 50 °C puis sous vide en ne dépassant pas 50 °C.

De cette manière, on obtient 200 g de bis-(di-n-propylacétate)-1,3 de glycéryle sous forme d'un liquide jaune pâle.

Rendement : 58 %
$n_D^{20}$ = 1,447 2
Spectre infra-rouge : OH large à 3 500 $cm^{-1}$
$CH_3$, $CH_2$ à 2 980 $cm^{-1}$ (F)
2 950 $cm^{-1}$ (F)

2 890 $cm^{-1}$ (F)
CO ester à 1 740 $cm^{-1}$ (F)

Exemple 5

Dans un ballon de 500 ml, on introduit 269 g (275 ml) de pyridine et 18,2 g (0,2 mole) de glycérol. Sous agitation, on ajoute 100 g (0,623 mole) de chlorure de méthyl-1 cyclohexylcarbonyle en environ 15 minutes. On chauffe progressivement le milieu réactionnel jusqu'à 65/70 °C en une heure et on l'y maintient pendant 3 heures. Après cette opération, on chauffe au reflux pendant 2 heures puis on refroidit. On élimine sous vide la pyridine en excès à une température de 60 °C.

On reprend la masse pâteuse dans 250 g d'eau distillée et on extrait avec deux fractions chacune de 250 ml d'éther éthylique. On recueille les extraits organiques et on les lave successivement avec de l'acide chlorhydrique dilué jusqu'à pH = 1, avec de l'eau, avec une solution aqueuse à 5 % de carbonate de sodium jusqu'à pH ⩾ 10 et finalement avec de l'eau jusqu'à neutralité. Après séchage sur sulfate de sodium, on chauffe le mélange jusqu'à siccité et on reprend l'huile obtenue dans du toluène. On passe sur colonne contenant 730 g de silice et on reprend dans environ 500 ml d'éther éthylique la fraction correspondant au triglycéride pur. On traite cette solution avec 3 g de charbon actif, on filtre et on chauffe la solution obtenue jusqu'à siccité à une température de 60-70 °C et sous une pression de 0,5-1 mm Hg.

De cette manière, on obtient 50 g de tri-(méthyl-1 cyclohexylcarboxylate) de glycéryle sous la forme d'un liquide jaunâtre légèrement visqueux.

Rendement : 54 %
$n_D^{17}$ = 1,481 2
Spectre infra-rouge (film) : $CH_2$ à 2 940 $cm^{-1}$ (F)
2 860 $cm^{-1}$ (m)
CO ester à 1 740 $cm^{-1}$ (F)
1 205 $cm^{-1}$ (F)
1 160 $cm^{-1}$ (F)

Exemple 6

Préparation du (di-n-propylacétate)-1 (méthyl-1 cyclohexylcarboxylate)-3 de glycéryle.

Dans un ballon parfaitement sec, on introduit 1 000 ml de pyridine et 218 g (1 mole) de (di-n-propylacétate)-1 de glycéryle.

Sous agitation, on ajoute progressivement, à température ambiante, 240,8 g (1,5 mole) de chlorure de méthyl-1 cyclohexylcarbonyle. Après cette opération, on chauffe le milieu à 40 °C pendant 4 heures, puis jusqu'à 70 °C pendant 6 heures. Après refroidissement, on hydrolyse le milieu réactionnel en ajoutant 5 000 ml d'eau distillée. On extrait le milieu avec trois fractions chacune de 1 000 ml d'éther éthylique. On recueille les fractions éthérées et on les lave successivement avec de l'eau distillée, une solution aqueuse à 2 % d'acide chlorhydrique et de nouveau avec de l'eau distillée. Après séchage sur sulfate de sodium, on élimine l'éther sous pression réduite tout en ne dépassant pas la température de 40 °C.

On passe l'huile obtenue, sur colonne de silice et on élue avec un mélange hexane/éther éthylique.

De cette manière, on obtient 88 g de (di-n-propylacétate)-1 (méthyl-1 cyclohexylcarboxylate)-3 de glycéryle.

$n_D^{21}$ = 1,460 8
Spectre infra-rouge (film) : OH à 3 500 $cm^{-1}$ (m, f)
C = 0 à 1 740 $cm^{-1}$ (F)

En utilisant le même procédé que décrit précédemment, on a préparé le composé suivant :

Composé

(Di-n-propylacétate)-1(méthyl-2 éthyl-2 hexanoate)-3 de glycéryle
$n_D^{21}$ = 1,449 0
Spectre infra-rouge (film) : OH à 3 500 $cm^{-1}$ (f)
$CH_3$, $CH_2$ à 2 970 $cm^{-1}$ (F)
2 940 $cm^{-1}$ (F)
2 880 $cm^{-1}$ (F)
CO ester à 1 730 $cm^{-1}$ (F)
1 150 $cm^{-1}$ (f)

Exemple 7

Préparation du (di-n-propylacétate)-1 bis (méthyl-1 cyclohexylcarboxylate)-2,3 de glycéryle

Ce composé est obtenu (199,2 g) en supplément au (di-n-propylacétate)-1 (méthyl-1 cyclohexylcarboxylate)-3 de glycéryle dans l'Exemple 6.

$n_D^{20}$ = 1,466 9

Spectre infra-rouge (film) : $CH_3$, $CH_2$ à 2 970 $cm^{-1}$
2 940 $cm^{-1}$ (F)
2 870 $cm^{-1}$ (F)
CO ester à 1 740 $cm^{-1}$ (F)
1 160 $cm^{-1}$ (m)

## Exemple 8

Préparation du (di-n-propylacétate)-1 (méthyl-1 cyclohexylcarboxylate)-2 de glycéryle

a) Di-n-propylacétate d'époxy-2,3 propyle

Dans un ballon, on introduit 70 g (0,48 mole) d'acide di-n-propylacétique, 250 ml de toluène et 30 ml de N,N-diméthylformamide. Après cette opération, on ajoute 40 g d'une solution aqueuse à 50 % d'hydroxide de sodium. On chauffe ce mélange à 60°-80 °C et on introduit 138,7 g (1,5 mole) d'épichlorhydrine.

On maintient le milieu réactionnel sous reflux pendant 6 heures à 70-80 °C puis on le concentre dans un évaporateur rotatif.

Après extraction avec l'éther éthylique, on recueille les fractions organiques et on distille sous 1 mm Hg en recueillant la fraction bouillant entre 76 et 78 °C.

De cette manière, on obtient le di-n-propylacétate d'époxy-2,3 propyle sous la forme d'un liquide limpide incolore.

$n_D^{21}$ = 1,435 1

Spectre infra-rouge (film) : $CH_3$, $CH_2$ à 2 975 $cm^{-1}$ (F)
2 950 $cm^{-1}$ (F)
2 890 $cm^{-1}$ (F)
CO ester à 1 745 $cm^{-1}$ (F)
1 180 $cm^{-1}$ (F)
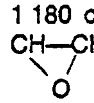 à 1 260 $cm^{-1}$ (m)

b) (Di-n-propylacétate)-1 (méthyl-1 cyclohexylcarboxylate)-2 de glycéryle

Dans un ballon, on chauffe à 70 °C pendant 90 minutes, 4 g (0,02 mole) de di-n-propylacétate d'époxy-2,3 propyle et 5,6 g (0,04 mole) d'acide méthyl-1 cyclohexylcarboxylique. Après cette opération, on ajoute quelques gouttes d'éthérate de trifluorure de bore. On laisse le mélange revenir à la température ambiante puis on extrait avec de l'éther éthylique. On lave les fractions organiques avec du carbonate de sodium et on évapore l'éther. On purifie ensuite le mélange de diesters-1,2 et -1,3 ainsi obtenu par chromatographie sur colonne de silice.

De cette manière, on obtient le (di-n-propylacétate)-1 (méthyl-1 cyclohexylcarboxylate)-2 de glycéryle.

## Exemple 9

Préparation d'un mélange de bis-(di-n-propylacétates)-1,2 et -1,3 de glycéryle (« Mélange A »)

Dans un ballon parfaitement sec, on introduit 9,1 kg (9,3 l) de pyridine et 1,299 kg (13,36 moles) de glycérol.

Sous vive agitation, on refroidit le mélange à 0/– 5 °C. Après quoi, on ajoute 4,340 kg (26,72 moles) de chlorure de di-n-propylacétyle tout en maintenant le milieu réactionnel inférieur à 10 °C. Cette opération nécessite 3 h 30 minutes. On laisse retourner le milieu à la température ambiante puis on l'y abandonne pendant environ 15 heures. On distille ensuite la pyridine sous pression réduite et sans dépasser 50 °C.

Après refroidissement, on ajoute 3,5 l d'eau et 3,5 l de benzène. On décante la phase aqueuse contenant le chlorhydrate de pyridine et on extrait à nouveau avec 3 l de benzène. On recueille les fractions benzéniques et on les lave successivement avec une solution aqueuse d'acide chlorhydrique à 36 % jusqu'à pH = 1 puis avec de l'eau jusqu'à neutralité.

On élimine le benzène par distillation sous vide sans dépasser 50 °C dans la masse. On rectifie l'ester brut par portions chacune de 700 à 800 g et on recueille la fraction bouillant entre 130 °C/0,5 mm Hg et 135 °C/0,1 mm Hg.

De cette manière, on obtient 3,420 kg d'un mélange de bis-(di-n-propylacétates)-1,2 et -1,3 de glycéryle (« Mélange A »).

Rendement : 78 %

$n_D^{20}$ = 1,446 1

Spectre infra-rouge (film) : OH large à 3 490 $cm^{-1}$
$CH_3$, $CH_2$ à 2 950 $cm^{-1}$ (F)
2 880 $cm^{-1}$ (F)
CO ester à 1 730 $cm^{-1}$ (F)
1 150 $cm^{-1}$ (F)
et larges

Le mélange ainsi obtenu contient :

— environ 90 % de (di-n-propylacétates)-1,2 et -1,3 de glycéryle, à savoir environ 70 % de diester-1,3 et environ 30 % de diester-1,2
— environ 7 % de (di-n-propylacétate)-1 de glycéryle
— environ 2 % de tri-(di-n-propylacetate) de glycéryle
— environ 1 % d'impuretés, à savoir du glycérol et de l'acide di-n-propylacétique.

Exemple 10

Préparation d'un mélange de bis-(di-n-propylacétates)-1,2 et -1,3 de glycéryle (« Mélange B »)

Dans un ballon de 2 l équipé d'un réfrigérant à reflux, on introduit 893 g (900 ml) de N,N-diméthylformamide, 77,4 g (0,6 mole) de dichloro-1,3 propanol-2 et 206,4 g (1,24 mole + 3 %) de di-n-propylacétate de sodium. Sous atmosphère d'azote et vive agitation, on porte le milieu à 100 °C et on l'y maintient pendant 8 heures. On distille sous pression réduite (≃ 20 mm Hg), la quantité maximum de solvant et on recueille 893 g de distillat.

Au résidu ainsi obtenu (≃ 350 g) on ajoute 300 g d'eau distillée et 537 g (620 ml) de toluène. On lave la phase organique successivement avec 300 g d'eau distillée, une solution de 30 g de carbonate de sodium dans 400 g d'eau distillée et finalement avec autant de fractions de 300 g d'eau distillée que nécessaire pour obtenir un pH neutre. Après séchage sur sulfate de sodium, on élimine le solvant sous pression réduite avec un évaporateur rotatif pour obtenir un mélange brut de produits finaux (≃ 201 g ; ≃ 97,3 %). On rectifie ce mélange sous haut vide et on recueille la fraction bouillant à 152 °C sous 0,2 mm Hg. De cette manière, on obtient un mélange de bis-(di-n-propylacétates)-1,2 et -1,3 de glycéryle (« Mélange B ») sous la forme d'un liquide incolore.

$n_D^{20}$ = 1,447 6

Bis-(di-n-propylacétate)-1,3 de glycéryle/Bis-(di-n-propylacétate)-1,2 de glycéryle = 1,54.

(Di-n-propylacétate)-1 de glycéryle : environ 1 %

Tri-(di-n-propylacétate) de glycéryle : non détecté (< 0,5 %)

Dichloro-1,3 propanol-2 : 0,002 %

Suivant le même procédé que décrit précédemment, on a préparé les mélanges suivants de diesters :

Mélanges

Bis-(di-n-propylpropionate)-1,2 de glycéryle/bis-(di-n-propylpropionate)-1,3 de glycéryle.

P.E. : 172-180 °C sous 0,4 mm Hg

$n_D^{23}$ = 1,456 7

Spectre infra-rouge (film) : OH à 3 480 $cm^{-1}$ (m)
C = O à 1 740 $cm^{-1}$ (F)
C—O à 1 170 $cm^{-1}$ (m)

Bis-(di-n-propylméthoxyacétate)-1,2 de glycéryle/bis-(di-n-propylméthoxy-acétate)-1,3 de glycéryle.

$n_D^{14}$ = 1,455 6

Spectre infra-rouge (film) : OH à 3 480 $cm^{-1}$ (m)
C = O à 1 755 $cm^{-1}$ (F)
COO à 1 100-1 200 $cm^{-1}$ (m)

D'autres essais ont été effectués en vue de préparer un mélange de bis-(di-n-propylacétates)-1,2 et -1,3 de glycéryle selon le procédé décrit ci-dessus tout en respectant les conditions opératoires suivantes :

(Voir tableau, p. 23)

| Estérification | | Excès de di-n-propylacétate de sodium (en %) | Diméthylformamide / Eau | Rendement (en %) |
|---|---|---|---|---|
| T (°C) | Temps (h) | | | |
| 110 | 9 | - | pas d'eau | 88,8 |
| 100 | 8 | 5* | pas d'eau | 93,9 |
| 100 | 5 | - | ≃ 95/5 | 90,7 |
| 100 | 7 | - | ≃ 95/5 | 94 |

* diméthylformamide utilisé en quantité équivalent à 5 fois la quantité totale des réactifs

Exemple 11

Suivant des techniques pharmaceutiques connues on a préparé une capsule contenant les ingrédients suivants :

| Ingrédient | mg |
|---|---|
| Mélange de bis-(di-n-propylacétates)-1,2 et -1,3 de glycéryle (« Mélange B ») | 500 |
| Gélatine | 212 |
| Glycérol | 83 |
| p-Hydroxybenzoate d'éthyle (sodé) | 4 |
| p-Hydroxybenzoate de propyle (sodé) | 2 |
| Dioxyde de titane | 4,4 |

**Revendications**

1. Mono-, di- et triesters du glycérol correspondant à la formule générale

$$\underset{OR}{\overset{|}{CH_2}} - \underset{OR_1}{\overset{|}{CH}} - \underset{OR_2}{\overset{|}{CH_2}}$$

dans laquelle R, $R_1$ et $R_2$, qui sont identiques ou différents, représentent chacun hydrogène ou un radical acyle à savoir di-n-propylacétyle, tri-n-propylacétyle, éthyl-2 hexanoyle, n-propyl-4 heptanoyle, n-propyl-5 octanoyle, méthyl-2 éthyl-2 hexanoyle, méthyl-1 cyclohexylcarbonyle ou di-n-propylméthoxyacétyle à condition que R, $R_1$ et $R_2$ ne soient pas simultanément hydrogène ou di-n-propylacétyle.

2. (Di-n-propylacétate)-1 de glycéryle.
3. (Tri-n-propylacétate)-1 de glycéryle.
4. (Ethyl-2 hexanoate)-1 de glycéryle.
5. (Méthyl-1 cyclohexylcarboxylate)-1 de glycéryle.
6. (Méthyl-2 éthyl-2 hexanoate)-1 de glycéryle.
7. (Di-n-propylacétate)-2 de glycéryle.
8. Bis-(di-n-propylacétate)-1,2 de glycéryle.
9. Bis-(éthyl-2 hexanoate)-1,2 de glycéryle.
10. Bis-(méthyl-1 cyclohexylcarboxylate)-1,2 de glycéryle.
11. Bis-(tri-n-propylacétate)-1,2 de glycéryle.
12. Bis-(méthyl-2 éthyl-2 hexanoate)-1,2 de glycéryle.
13. Bis-(di-n-propylacétate)-1,3 de glycéryle.
14. (Di-n-propylacétate)-1 (méthyl-1 cyclohexylcarboxylate)-3 de glycéryle.
15. (Di-n-propylacétate)-1 bis-(méthyl-1 cyclohexylcarboxylate)-2,3 de glycéryle.
16. (Di-n-propylacétate)-1 (méthyl-2 éthyl-2 hexanoate)-3 de glycéryle.
17. (Di-n-propylacétate)-1 (méthyl-1 cyclohexylcarboxylate)-2 de glycéryle.
18. Tri-(méthyl-1 cyclohexylcarboxylate)-1,2,3 de glycéryle.
19. (Di-n-propylméthoxyacétate)-1 de glycéryle.
20. (n-Propyl-4 heptanoate)-1 de glycéryle.
21. (n-Propyl-5 octanoate)-1 de glycéryle.
22. Mélange de bis(di-n-propylacétate)-1,3 de glycéryle/bis-(di-n-propylacétate)-1,2 de glycéryle

dans un rapport de 1,43 : 1 à 1,54 : 1.

23. Composition pharmaceutique ou vétérinaire comprenant comme principe actif essentiel, au moins un mono-, di- ou triester selon l'une quelconque des revendications 1 à 21, en association avec un véhicule pharmaceutique ou un excipient approprié.

24. Composition pharmaceutique ou vétérinaire comprenant comme principe actif essentiel un mélange de bis-(di-n-propylacétate)-1,3 de glycéryle/bis-(di-n-propylacétate)-1,2 de glycéryle selon la revendication 22, en association avec un véhicule pharmaceutique ou un excipient approprié.

25. Procédé de préparation de diesters-1,2 du glycérol selon la revendication 1 dans laquelle R représente hydrogène et $R_1$ et $R_2$ sont identiques et représentent un radical acyle caractérisé en ce que l'on fait réagir un diester de formule générale

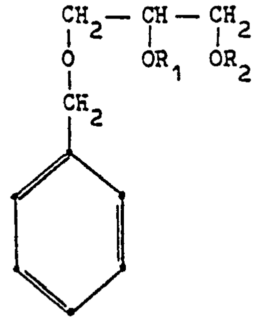

dans laquelle $R_1$ et $R_2$ sont identiques et représentent un radical acyle défini précédemment, avec l'hydrogène en milieu alcoolique et en présence de charbon palladié comme catalyseur, la réaction étant effectuée à la pression atmosphérique et à température ambiante.

26. Procédé de préparation de diesters-1,3 du glycérol selon la revendication 1 dans laquelle R et $R_2$ sont identiques et représentent un radical acyle et $R_1$ représente hydrogène, caractérisé en ce que l'on réduit, à température ambiante, un cétodiester de formule générale

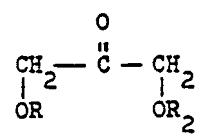

dans laquelle R et $R_2$ sont identiques et représentent un radical acyle défini précédemment, avec le borohydrure de potassium dans un éther puis on hydrolyse le complexe ainsi formé avec un acide fort inorganique.

27. Procédé de préparation de mélanges de diesters-1,2 et diester-1,3 du glycérol correspondant à la formule générale

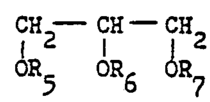

dans laquelle $R_5$ représente un radical di-n-propylacétyle, di-n-propylméthoxyacétyle ou di-n-propylpropionyle et $R_6$ et $R_7$, qui sont différents, représentent chacun hydrogène ou un radical di-n-propylacétyle, di-n-propylméthoxyacétyle ou di-n-propylpropionyle, $R_5$ et $R_6$ ou $R_5$ et $R_7$ étant identiques, caractérisé en ce que l'on estérifie, dans le N,N-diméthylformamide comme solvant, un équivalent de dichloro-1,3 propanol-2, à une température comprise entre 100 et 110 °C et pendant 5 à 10 heures, avec deux équivalents, de préférence en excès, d'un sel de métal alcalin d'acide di-n-propylacétique, d'acide di-n-propylméthoxyacétique ou d'acide di-n-propylpropionique puis on rectifie le mélange d'esters ainsi obtenu.

28. Procédé de préparation de mélanges de bis-(di-n-propylacétate)-1,3 de glycéryle/bis-(di-n-propylacétate)-1,2 de glycéryle dans un rapport de 1,43 à 1,54, caractérisé en ce que l'on estérifie, dans le N,N-diméthylformamide comme solvant, un équivalent de dichloro-1,3 propanol-2, à une température comprise entre 100 et 110 °C et pendant 5 à 10 heures, avec deux équivalents, de préférence en excès, d'un sel de métal alcalin d'acide di-n-propylacétique puis on rectifie le mélange d'esters ainsi obtenu.

29. Procédé selon la revendication 27 ou 28 caractérisé en ce que le sel de métal alcalin est le sel de sodium.

30. Procédé selon la revendication 27 ou 28 caractérisé en ce que le sel de métal alcalin est en excès de 5 % maximum.

31. Procédé selon la revendication 27 ou 28 caractérisé en ce que la température est 100 °C et l'estérification est effectuée pendant 8 heures.

32. Mélange de bis-(di-n-propylacétate)-1,3 de glycéryle/bis-(di-n-propylacétate)-1,2 de glycéryle obtenu en application du procédé selon l'une quelconque des revendications 27 à 31.

## Claims

1. Glycerol mono-, di- and tri-esters corresponding to the general formula

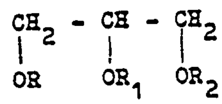

in which R, $R_1$ and $R_2$, which are the same or different, each represent hydrogen or an acyl radical namely di-n-propylacetyl, tri-n-propylacetyl, 2-ethyl-hexanoyl, 4-n-propyl-heptanoyl, 5-n-propyl-octanoyl, 2-methyl-2-ethyl-hexanoyl, 1-methyl-cyclohexylcarbonyl or di-n-propylmethoxyacetyl with the proviso that R, $R_1$ and $R_2$ are not simultaneously hydrogen or di-n-propylacetyl.

2. Glycéryl 1-(di-n-propylacetate).

3. Glyceryl 1-(tri-n-propylacetate).

4. Glyceryl 1-(2-ethyl-hexanoate).

5. Glyceryl 1-(1-methyl-cyclohexylcarboxylate).

6. Glyceryl 1-(2-methyl-2-ethyl-hexanoate).

7. Glyceryl 2-(di-n-propylacetate).

8. Glyceryl 1,2-bis-(di-n-propylacetate).

9. Glyceryl 1,2-bis-(2-ethyl-hexanoate).

10. Glyceryl 1,2-bis-(1-methyl-cyclohexylcarboxylate).

11. Glyceryl 1,2-bis-(tri-n-propylacetate).

12. Glyceryl 1,2-bis-(2-methyl-2-ethyl-hexanoate).

13. Glyceryl 1,3-bis-(di-n-propylacetate).

14. Glyceryl 1-(di-n-propylacetate)-3-(1-methyl-cyclohexylcarboxylate).

15. Glyceryl 1-(di-n-propylacetate)-2,3-bis-(1-methyl-cyclohexylcarboxylate).

16. Glyceryl 1-(di-n-propylacetate)-3-(2-methyl-2-ethyl-hexanoate).

17. Glyceryl 1-(di-n-propylacetate)-2-(1-methyl-cyclohexylcárboxylate).

18. Glyceryl 1,2,3-tri-(1-methyl-cyclohexylcarboxylate).

19. Glyceryl 1-(di-n-propylmethoxyacetate).

20. Glyceryl 1-(4-n-propyl-heptanoate).

21. Glyceryl 1-(5-n-propyl-octanoate).

22. Mixture of glyceryl 1,3-bis-(di-n-propylacetate)/glyceryl 1,2-bis-(di-n-propylacetate) in a ratio of 1.43 : 1 to 1.54 : 1.

23. A pharmaceutical or veterinary composition comprising as essential active ingredient at least one mono-, di- or tri-ester according to any of Claims 1 to 21, in association with a pharmaceutical carrier or excipient therefor.

24. A pharmaceutical or veterinary composition comprising as essential active ingredient a mixture of glyceryl 1,3-bis-(di-n-propylacetate)/glyceryl 1,2-bis-(di-n-propylacetate) according to Claim 22, in association with a pharmaceutical carrier or excipient therefor.

25. Process for preparing glycerol 1,2-diesters according to Claim 1 in which R represents hydrogen and $R_1$ and $R_2$ are identical and represent an acyl radical whereby a diester of general formula

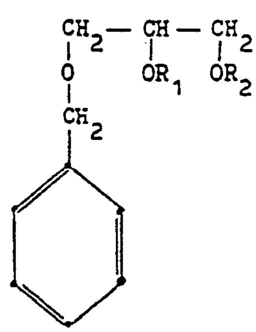

in which $R_1$ and $R_2$ are identical and represent an acyl radical as defined above, is reacted with hydrogen in an alcoholic medium and in the presence of palladium charcoal as catalyst, the reaction being conducted at atmospheric pressure and at room-temperature.

26. Process for preparing glycerol 1,3-diesters according to Claim 1 in which R and $R_2$ are identical

and represent an acyl radical and $R_1$ represents hydrogen, whereby a ketodiester of general formula

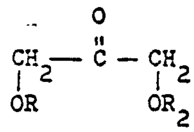

in which R and $R_2$ are identical and represent an acyl as defined above, is reduced with potassium borohydride in an ether and the complex so formed is subsequently hydrolysed with a strong inorganic acid.

27. Process for preparing mixtures of glycerol 1,2-diesters and 1,3-diesters corresponding to the general formula

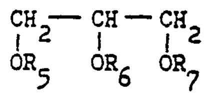

in which $R_5$ represents a di-n-propylacetyl, di-n-propylmethoxyacetyl or di-n-propylpropionyl radical and $R_6$ and $R_7$, which are different, each represent hydrogen or a di-n-propylacetyl, di-n-propylmethoxyacetyl or di-n-propylpropionyl radical, $R_5$ and $R_6$ or $R_5$ and $R_7$ being identical, whereby one equivalent of 1,3-dichloro-2-propanol is esterified at a temperature between 100 and 110 °C and for 5 to 10 hours with two equivalents, preferably in excess, of an alkali metal salt of di-n-propylacetic acid, di-n-propylmethoxyacetic acid or di-n-propylpropionic acid in N,N-dimethylformamide as solvent followed by rectification of the mixture of esters so obtained.

28. Process for preparing mixtures of glyceryl 1,3-bis-(di-n-propylacetate)/1,2-bis-(di-n-propylacetate) in a ratio of 1.43 to 1.54, whereby one equivalent of 1,3-dichloro-2-propanol is esterified at a temperature between 100 and 110 °C and for 5 to 10 hours with two equivalents, preferably in excess, of an alkali metal salt of di-n-propylacetic acid in N,N-dimethylformamide as solvent followed by rectification of the mixture of esters so obtained.

29. Process according to Claim 27 or 28 wherein the alkali metal salt is the sodium salt.

30. Process according to Claim 27 or 28 wherein the alkali metal salt is in excess of a maximum of 5 %.

31. Process according to Claim 27 or 28 wherein the temperature is 100 °C and the time of esterification is 8 hours.

32. Mixture of glyceryl 1,3-bis-(di-n-propylacetate)/glyceryl 1,2-bis-(di-n-propylacetate) obtained by application of the process in accordance with any of Claims 27 to 31.

**Ansprüche**

1. Glycerin-mono-, -di- und -triester der allgemeinen Formel

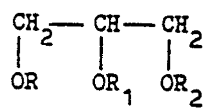

in der R, $R_1$ und $R_2$ gleich oder unterschiedlich jeweils Wasserstoff oder einen Acylrest, nämlich Di-n-propylacetyl, Tri-n-propylacetyl, 2-Ethyl-hexanoyl, 4-n-Propylheptanoyl, 5-n-Propyl-octanoyl, 2-Methyl-2-ethyl-hexanoyl, 1-Methyl-cyclohexylcarbonyl oder Di-n-propylmethoxyacetyl bedeuten, mit der Maßgabe, daß R, $R_1$ und $R_2$ nicht gleichzeitig Wasserstoff oder Di-n-propylacetyl sind.

2. Glyceryl-1-(di-n-propylacetat).
3. Glyceryl-1-(tri-n-propylacetat).
4. Glyceryl-1-(2-ethyl-hexanoat).
5. Glyceryl-1-(1-methyl-cyclohexylcarboxylat).
6. Glyceryl-1-(2-methyl-2-ethyl-hexanoat).
7. Glyceryl-2-(di-n-propylacetat).
8. Glyceryl-1,2-bis-(di-n-propylacetat).
9. Glyceryl-1,2-bis-(2-ethyl-hexanoat).
10. Glyceryl-1,2-bis-(1-methyl-cyclohexylcarboxylat).
11. Glyceryl-1,2-bis-(tri-n-propylacetat).
12. Glyceryl-1,2-bis-(2-methyl-2-ethyl-hexanoat).
13. Glyceryl-1,3-bis-(di-n-propylacetat).
14. Glyceryl-1-(di-n-propylacetat)-3-(1-methyl-cyclohexylcarboxylat).

15. Glyceryl-1-(di-n-propylacetat)-2,3-bis-(1-methyl-cyclohexylcarboxylat).
16. Glyceryl-1-(di-n-propylacetat)-3-(2-methyl-2-ethylhexanoat).
17. Glyceryl-1-(di-n-propylacetat)-2-(1-methyl-cyclohexylcarboxylat).
18. Glyceryl-1,2,3-tri-(1-methyl-cyclohexylcarboxylat).
19. Glyceryl-1-(di-n-propylmethoxyacetat).
20. Glyceryl-1-(4-n-propyl-heptanoat).
21. Glyceryl-1-(5-n-propyl-octanoat).
22. Gemisch von Glyceryl-1,3-bis-(di-n-propylacetat)/Glyceryl-1,2-bis-(di-n-propylacetat) in einem Verhältnis von 1,43 : 1 bis 1,54 : 1.

23. Eine pharmazeutische oder veterinäre Zusammensetzung, umfassend als essentiellen Wirkstoff mindestens einen Mono-, Di- oder Triester nach irgendeinem der Ansprüche 1-21 in Assoziation mit einem pharmazeutischen Träger oder Exzipienten dafür.

24. Eine pharmazeutische oder veterinäre Zusammensetzung, umfassend als essentiellen Wirkstoff ein Gemisch von Glyceryl-1,3-bis-(di-n-propylacetat)/Glyceryl-1,2-bis-(di-n-propylacetat) nach Anspruch 22 in Assoziation mit einem pharmazeutischen Träger oder Exzipienten dafür.

25. Verfahren zur Herstellung von Glycerin-1,2-diestern nach Anspruch 1, bei denen R Wasserstoff bedeutet und $R_1$ und $R_2$ identisch sind und einen Acylrest bedeuten, wobei ein Diester der allgemeinen Formel

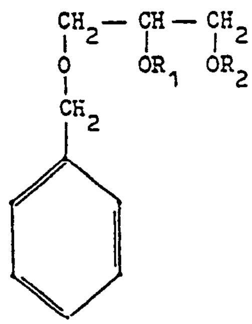

in der $R_1$ und $R_2$ identisch sind und einen vorstehend definierten Acylrest bedeuten, mit Wasserstoff in einem alkoholischen Medium in Gegenwart von Palladium-Holzkohle als Katalysator umgesetzt wird, wobei die Reaktion bei Atmosphärendruck und Raumtemperatur durchgeführt wird.

26. Verfahren zur Herstellung von Glycerin-1,3-diestern nach Anspruch 1, bei denen R und $R_2$ identisch sind und einen Acylrest bedeuten und $R_1$ Wasserstoff bedeutet, wobei ein Ketodiester der allgemeinen Formel

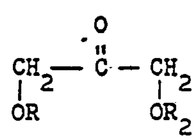

in der R und $R_2$ identisch sind und einen vorstehend definierten Acylrest bedeuten, mit Kaliumborhydrid in einem Ether reduziert wird und der gebildete Komplex anschließend mit einer starken anorganischen Säure hydrolysiert wird.

27. Verfahren zur Herstellung von Gemischen von Glycerin-1,2-diestern und -1,3-diestern der allgemeinen Formel

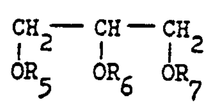

in der $R_5$ einen Di-n-propylacetyl-, Di-n-propylmethoxyacetat- oder Di-n-propylpropionylrest bedeutet und $R_6$ und $R_7$ unterschiedlich sind und jeweils Wasserstoff oder einen Di-n-propylacetyl-, Di-n-propylmethoxyacetyl- oder Di-n-propylpropionylrest bedeuten.und $R_5$ und $R_6$ oder $R_5$ und $R_7$ identisch sind, wobei 1 Äquivalent 1,3-Dichlor -2-propanol bei einer Temperatur zwischen 100 und 110 °C 5 bis 10 Stunden lang mit 2 Äquivalenten, vorzugsweise einem Überschuß, eines Alkalimetallsalzes von Di-n-propylessigsäure, Di-n-propylmethoxyessigsäure und Di-n-propylpropionsäure in N,N-Dimethylformamid als Lösungsmittel verestert wird, worauf das erhaltene Estergemisch rektifiziert wird.

28. Verfahren zur Herstellung von Gemischen von Glyceryl-1,3-bis-(di-n-propylacetat)/-1,2-bis-(di-n-propylacetat) in einem Verhältnis von 1,43 bis 1,54, wobei 1 Äquivalent 1,3-Dichlor-2-propanol bei einer Temperatur zwischen 100 und 110 °C 5 bis 10 Stunden lang mit 2 Äquivalenten, vorzugsweise einem Überschuß, eines Alkalimetallsalzes von Di-n-propylessigsäure in N,N-Dimethylformamid als Lösungsmittel verestert wird, worauf das erhaltene Estergemisch rektifiziert wird.

29. Verfahren nach Anspruch 27 oder 28, worin das Alkalimetallsalz das Natriumsalz ist.

30. Verfahren nach Anspruch 27 oder 28, worin das Alkalimetallsalz in einem Überschuß von maximal 5 % vorliegt.

31. Verfahren nach Anspruch 27 oder 28, worin die Temperatur 100 °C und die Veresterungszeit 8 Stunden beträgt.

32. Gemisch von Glyceryl-1,3-bis-(di-n-propylacetat)/Glyceryl-1,2-bis-(di-n-propylacetat), erhalten durch Anwendung des Verfahrens nach irgendeinem der Ansprüche 27-31.

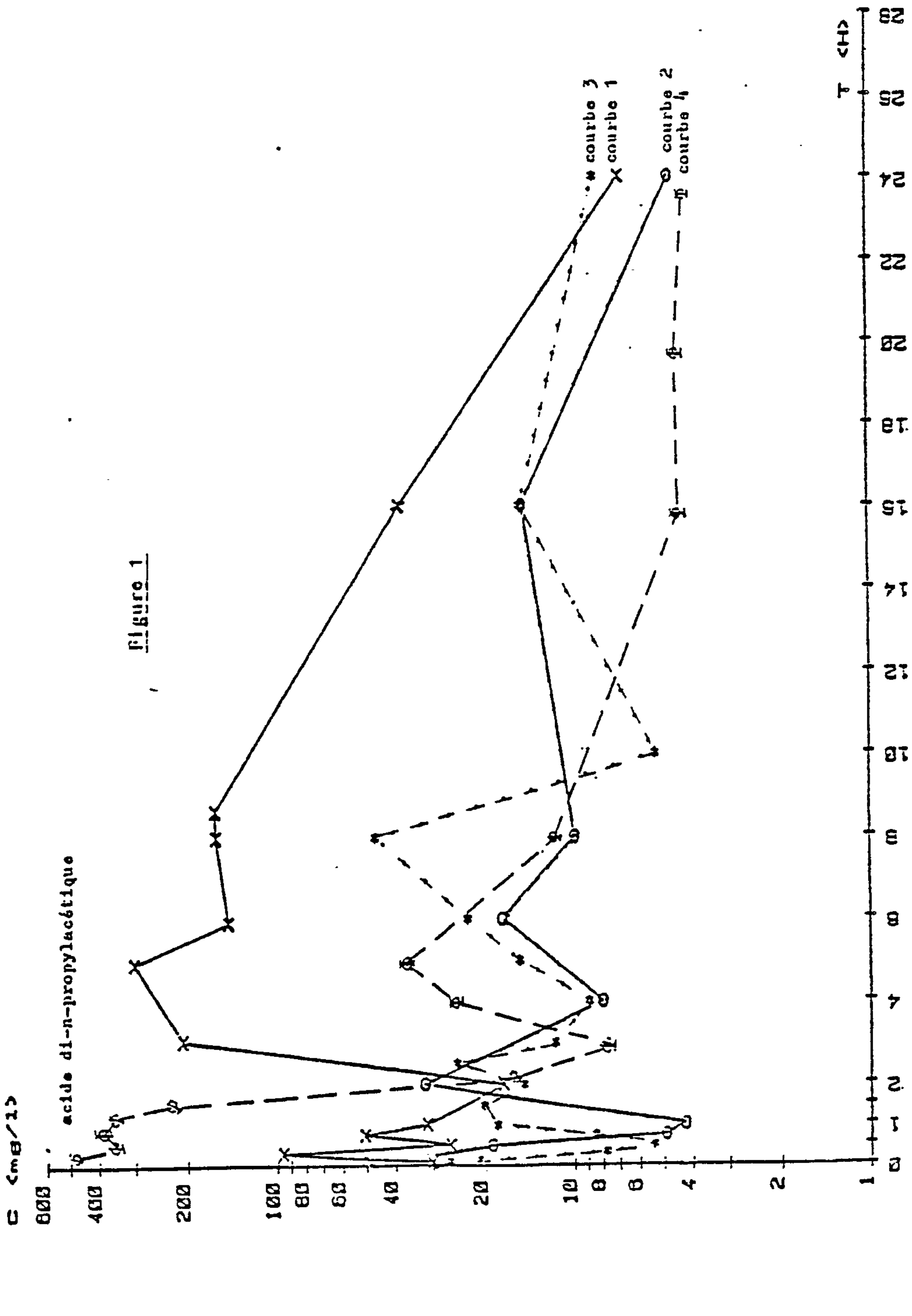
Figure 1
acide di-n-propylacétique
C <mg/l>
T <H>
courbe 1
courbe 2
courbe 3
courbe 4
600
400
200
100
80
60
40
20
10
8
6
4
2
1
0
1
2
4
6
8
10
12
14
16
18
20
22
24
26
28

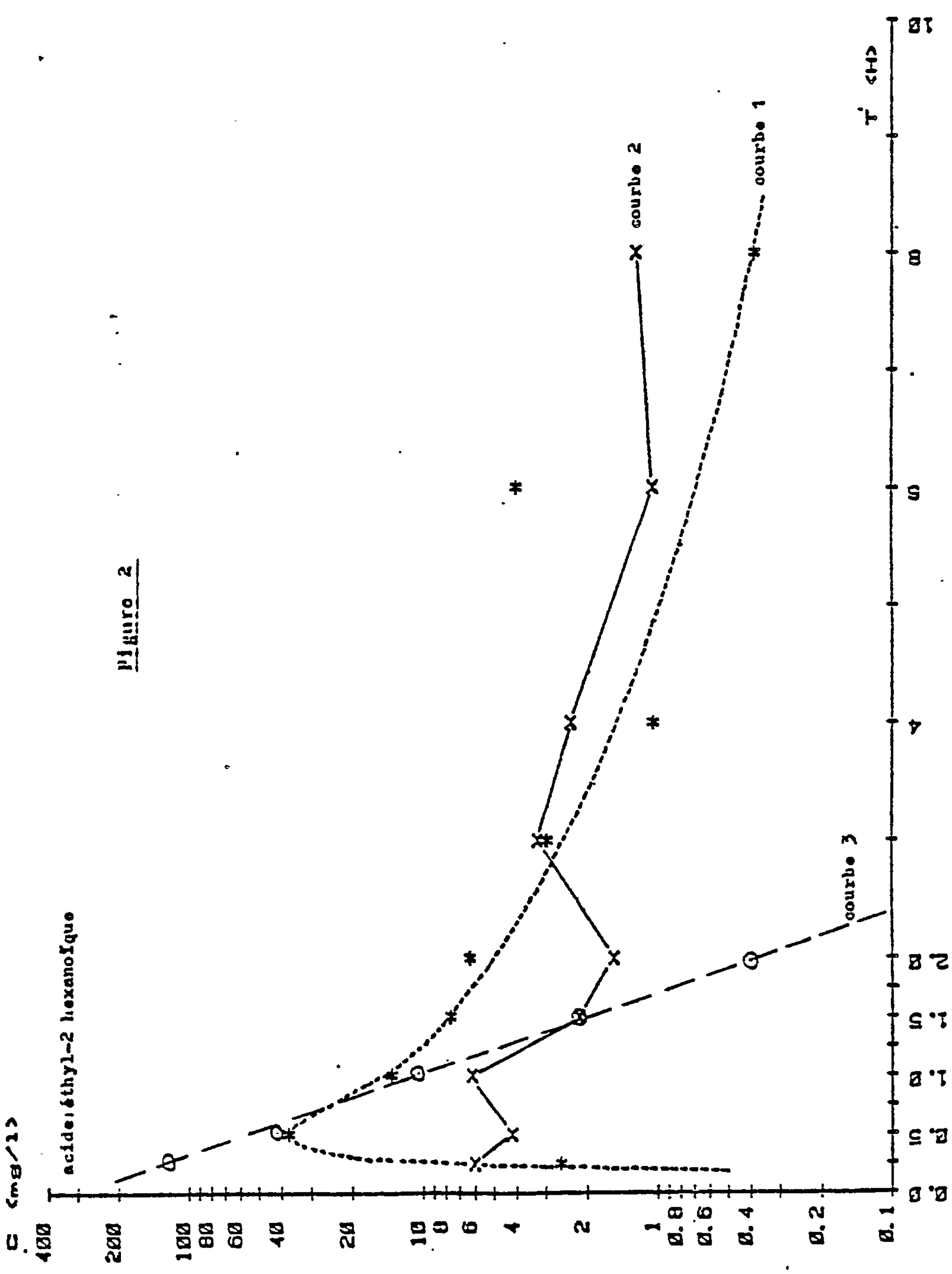
acide éthyl-2 hexanoïque
C (mg/l)
400
200
100
80
60
40
20
10
8
6
4
2
1
0.8
0.6
0.4
0.2
0.1
0.0
0.5
1.0
1.5
2.0
4
6
8
10
T (H)
courbe 1
courbe 2
courbe 3

Figure 2

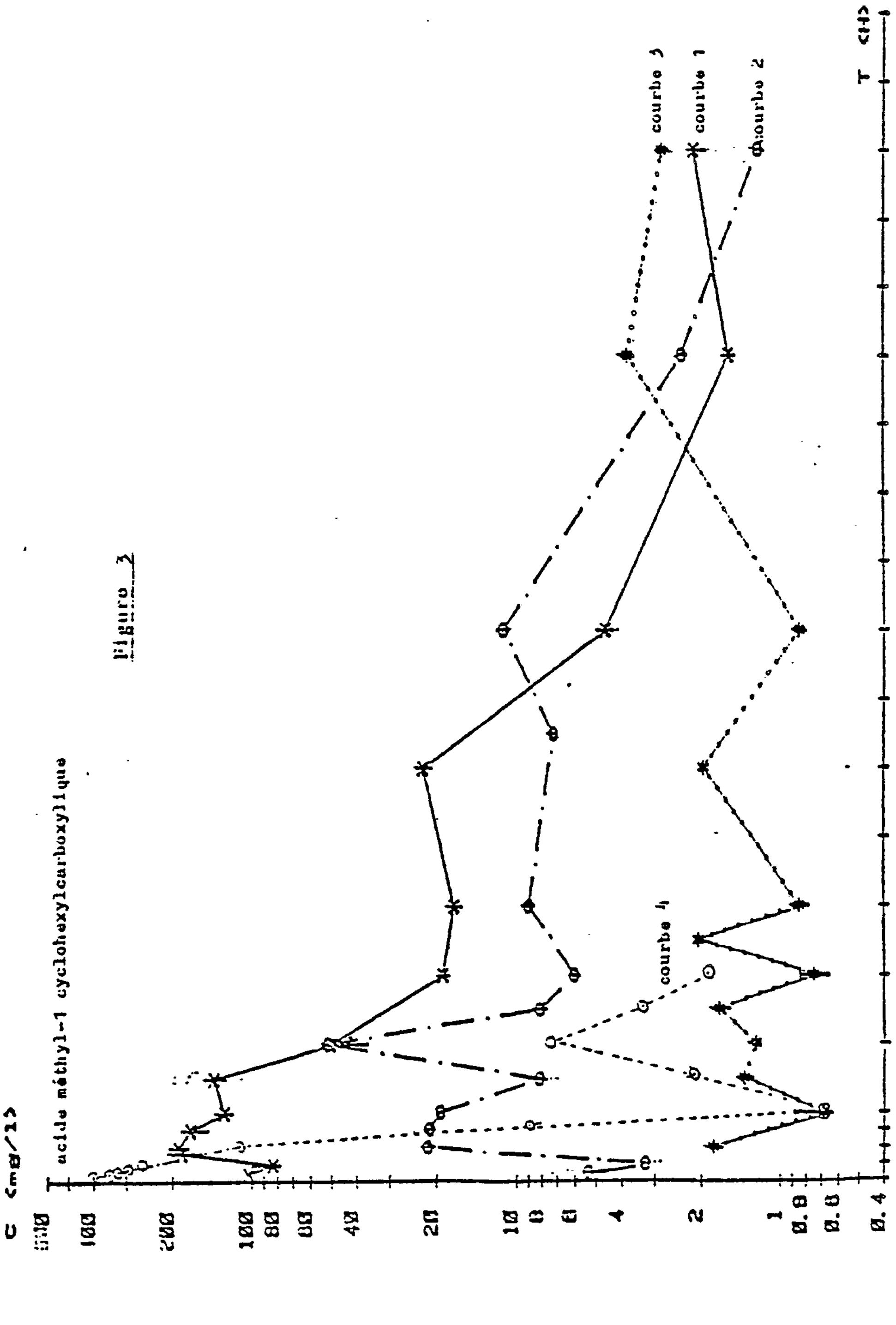
Figure 3
acide méthyl-1 cyclohexylcarboxylique
C <mg/l>
T <H>
courbe 1
courbe 2
courbe 3
courbe 4

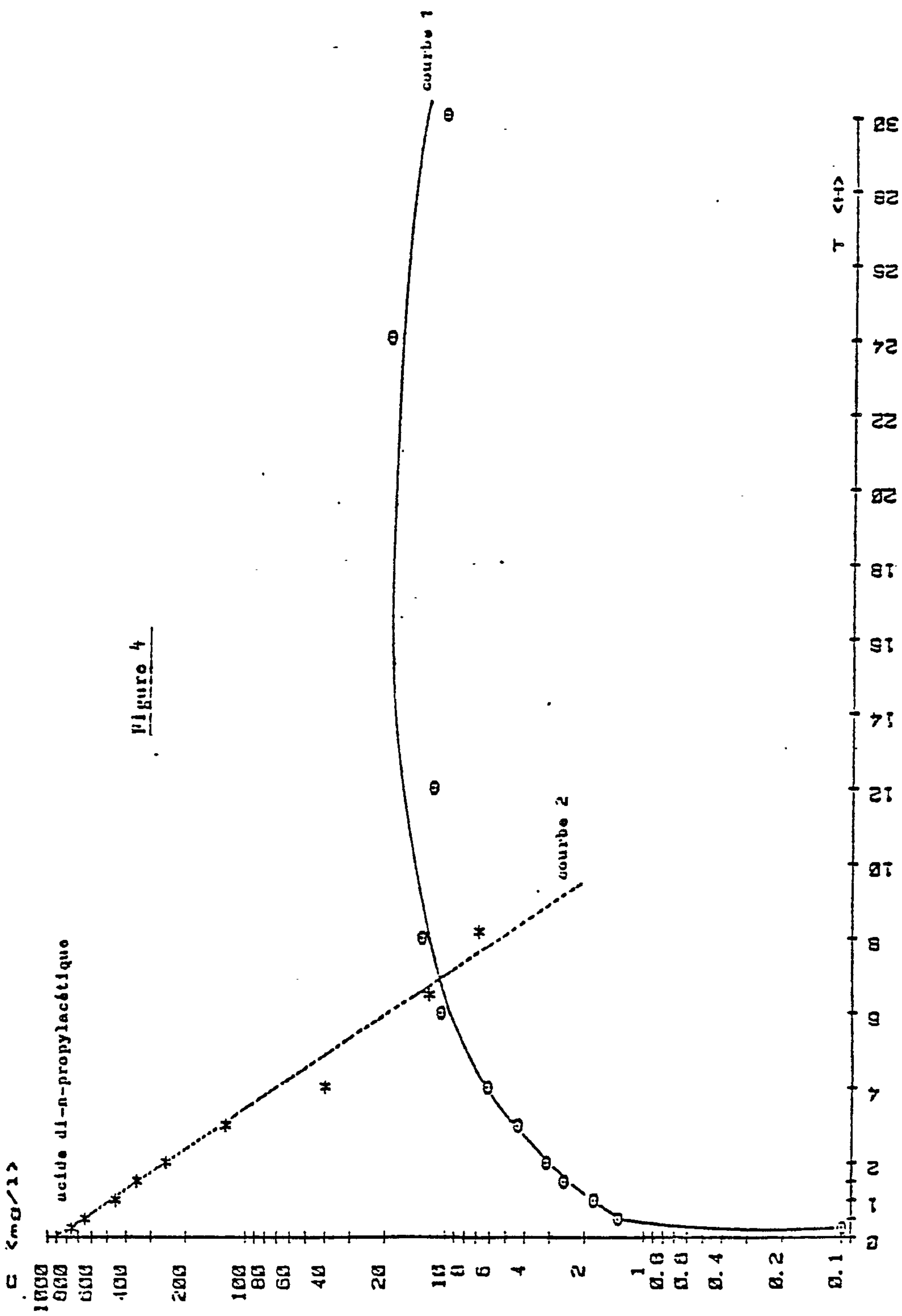
C (mg/l)
acide di-n-propylacétique
courbe 1
courbe 2
T (H)
1000
800
600
400
200
100
80
60
40
20
10
8
6
4
2
1
0.8
0.6
0.4
0.2
0.1

Figure 4

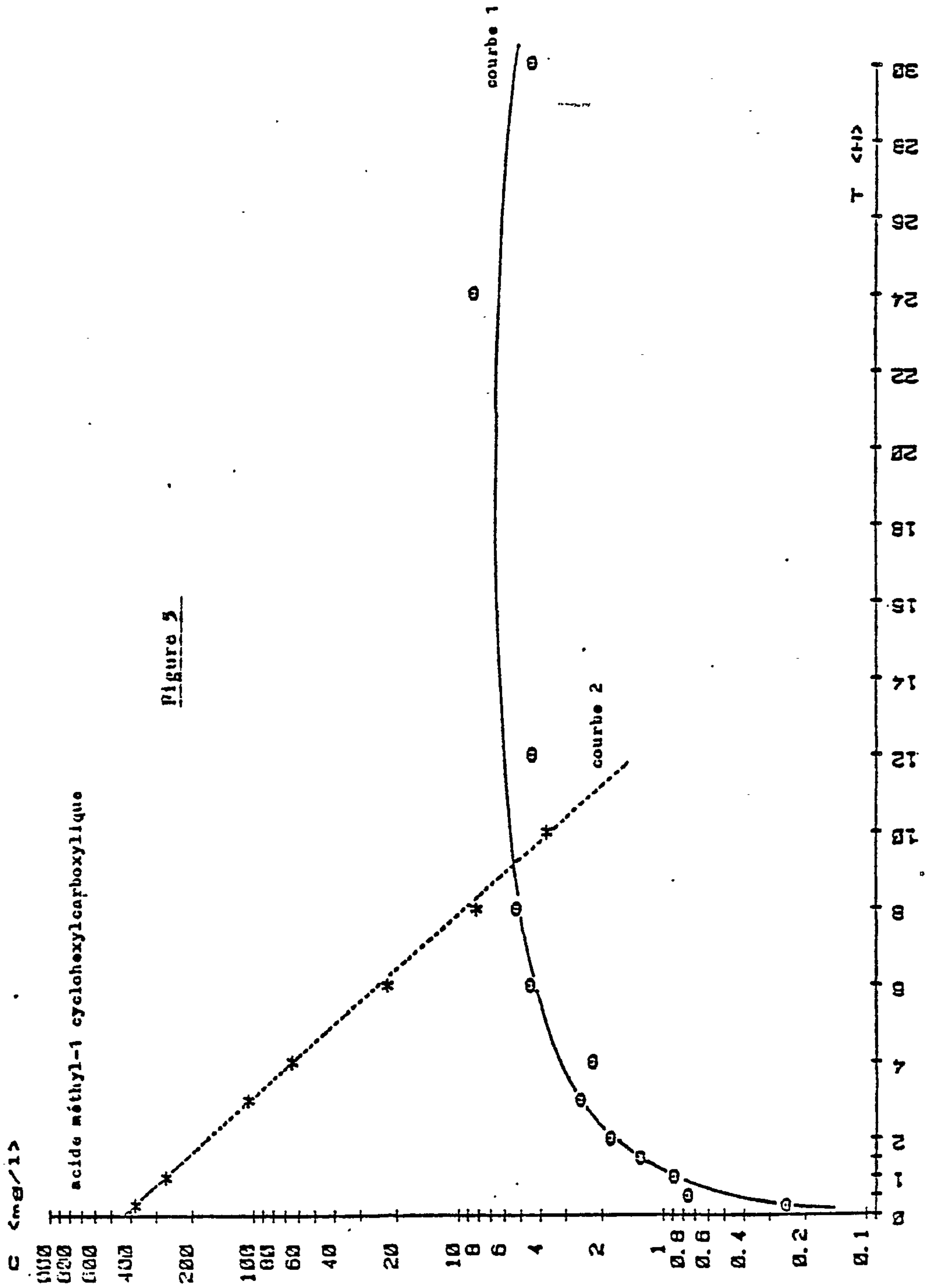
Figure 5
acide méthyl-1 cyclohexylcarboxylique
courbe 1
courbe 2
C (mg/l)
T (H)

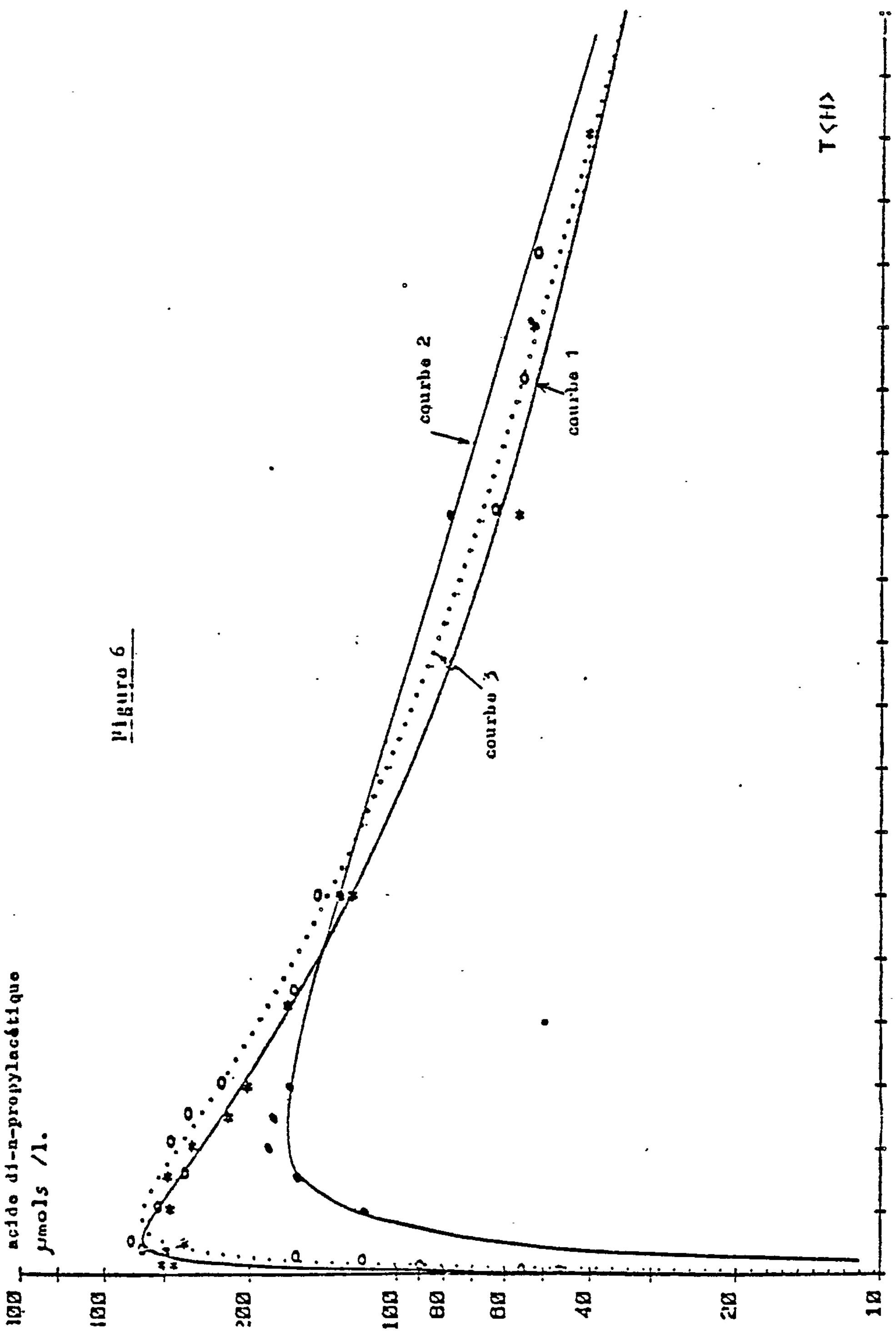
acide di-n-propylacétique
µmols /l.
courbe 1
courbe 2
courbe 3
T (H)

Figure 6

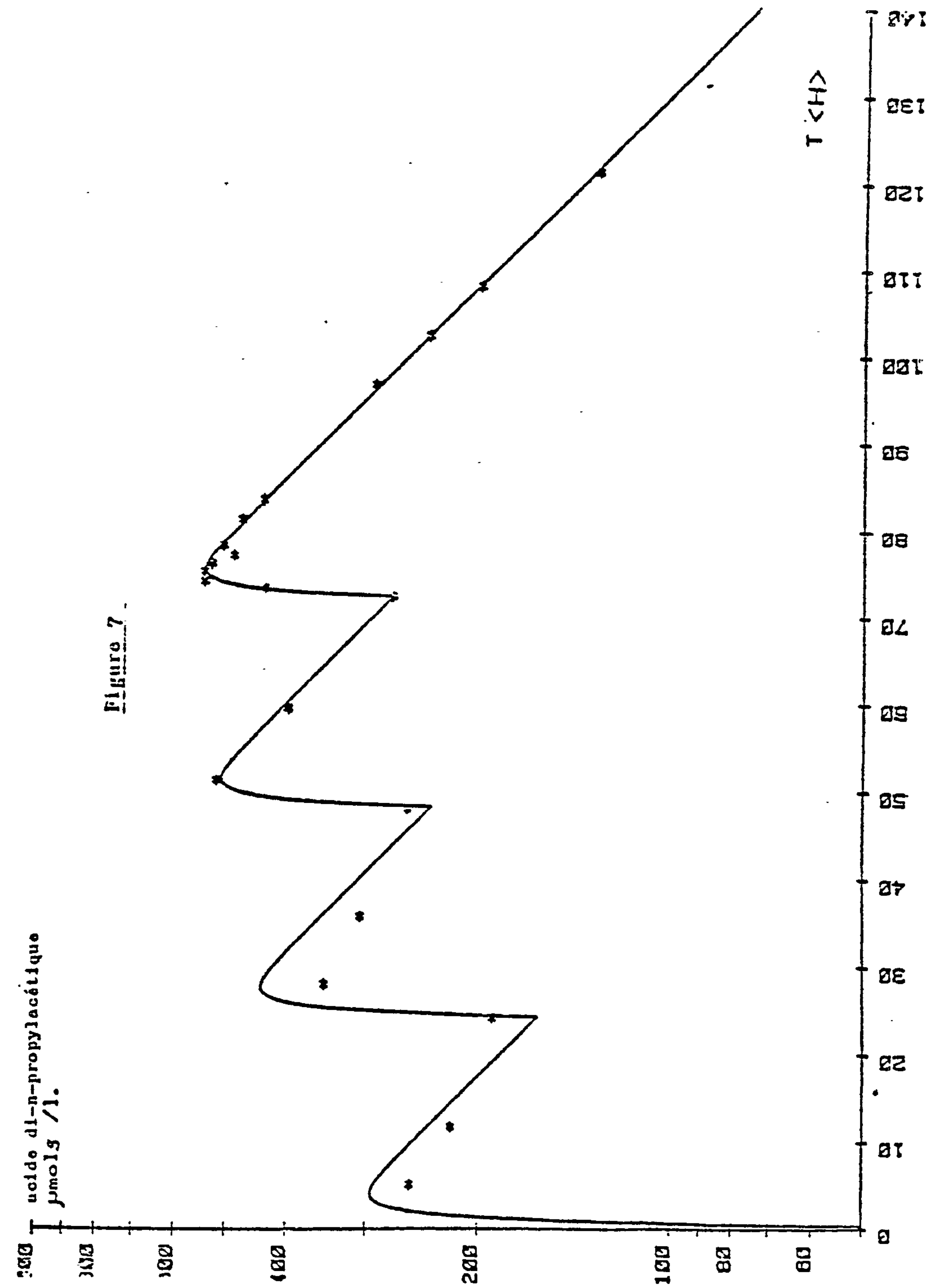


Figure 7

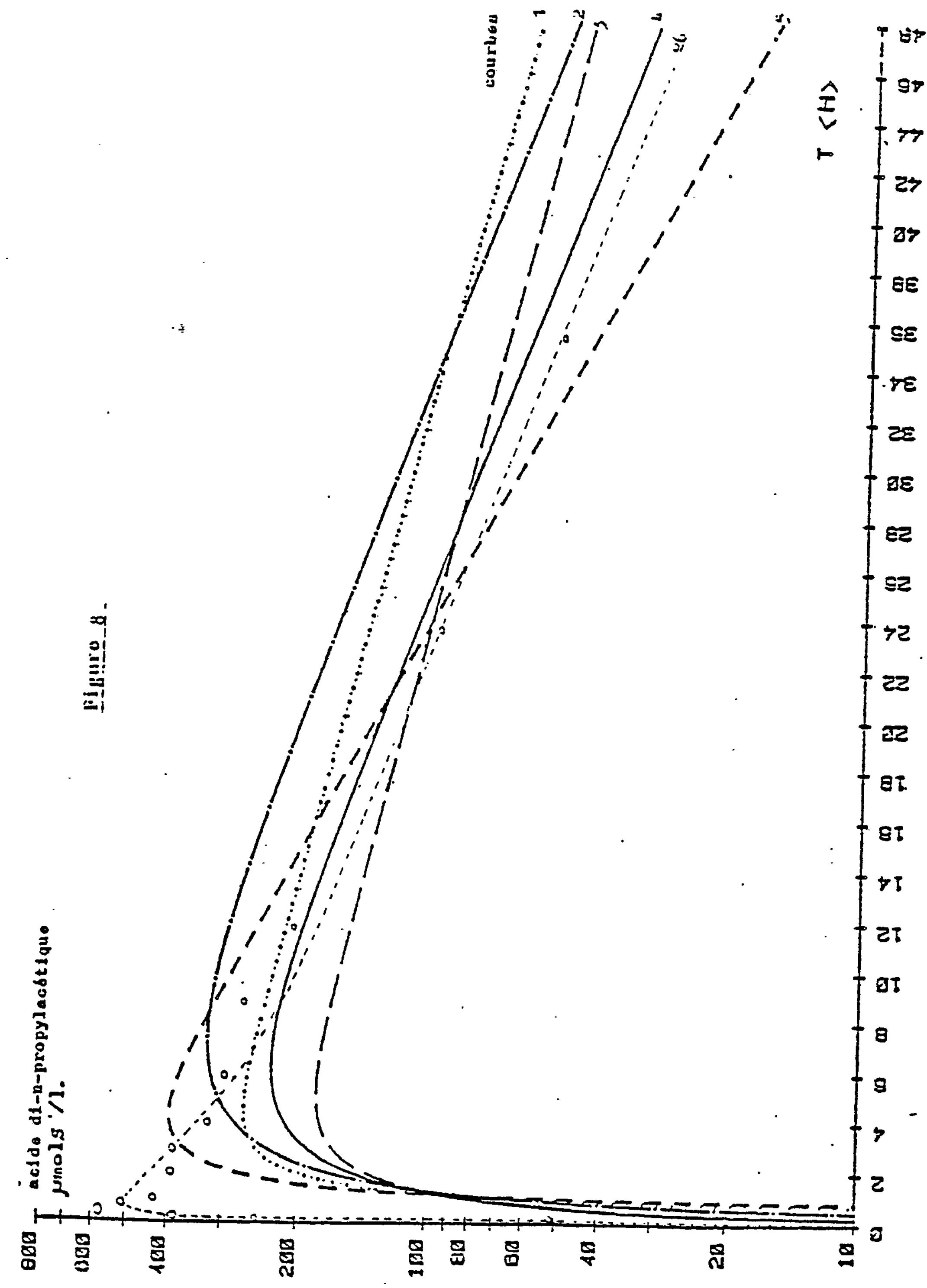
Figure 8
acide di-n-propylacétique
µmols/l.
800
600
400
200
100
80
60
40
20
10
T (H)
0
2
4
6
8
10
12
14
16
18
20
22
24
26
28
30
32
34
36
38
40
42
44
46
48
courbe
1
2
3
4
5
6